(19) Europäisches Patentamt / European Patent Office / Office européen des brevets

(11) **EP 4 197 605 A1**

(12) **EUROPEAN PATENT APPLICATION**
published in accordance with Art. 153(4) EPC

(43) Date of publication:
**21.06.2023 Bulletin 2023/25**

(21) Application number: **21863637.1**

(22) Date of filing: **01.09.2021**

(51) International Patent Classification (IPC):
**A63B 69/36** (2006.01)

(52) Cooperative Patent Classification (CPC):
**A63B 69/36; A63B 71/06**

(86) International application number:
**PCT/CN2021/116068**

(87) International publication number:
**WO 2022/048573 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.09.2020 CN 202010910396**

(71) Applicant: **Huawei Technologies Co., Ltd.**
**Shenzhen, Guangdong 518129 (CN)**

(72) Inventors:
• XU, Teng
  Shenzhen, Guangdong 518129 (CN)
• CHEN, Xiaohan
  Shenzhen, Guangdong 518129 (CN)
• JIANG, Yonghang
  Shenzhen, Guangdong 518129 (CN)

(74) Representative: **Gill Jennings & Every LLP**
**The Broadgate Tower**
**20 Primrose Street**
**London EC2A 2ES (GB)**

(54) **SWING MOTION DETECTION METHOD AND WEARABLE DEVICE**

(57) This application provides a swing action detection method and a wearable device. The method is applied to a wearable device, and the wearable device includes one or more motion sensors and a sound collector. In the method, the wearable device can collect a first sound signal by using the sound collector, to determine whether a user strikes a ball. After a strike action is determined, an exercise parameter of the user is obtained with reference to first exercise data collected by the wearable device by using the one or more motion sensors, so as to monitor the strike action of the user, and effectively assist the user in improving a strike rhythm and strike stability.

FIG. 23

EP 4 197 605 A1

**Description**

This application claims priority to Chinese Patent Application No. 202010910396.9, filed with the China National Intellectual Property Administration on September 2, 2020 and entitled "SWING ACTION DETECTION METHOD AND WEARABLE DEVICE", which is incorporated herein by reference in its entirety.

**TECHNICAL FIELD**

This application relates to the field of terminal technologies, and in particular, to a swing action detection method and a wearable device.

**BACKGROUND**

There are various types of ball-and-stick sports, and actual scenarios are complex. During ball-and-stick sports, a focus is mainly on key technologies such as club gripping, a strike preparation posture, and strike strength. For a beginner, the most important part of ball-and-stick sports is a strike action, and a strike speed directly affects a strike distance.

Currently, most ball-and-stick sports detection apparatuses on the market detect and classify swing actions based on radar waves by using a video analysis method. Installation of the apparatuses is complex, and high requirements are imposed on an installation space size and an operating environment. On a cloudy day or in a dark place, measurement errors of the apparatuses are large.

**SUMMARY**

This application provides a swing action detection method and a wearable device. The wearable device collects a sound signal by using a sound collector, to determine whether a user strikes a ball. In addition, a swing action parameter of the user is obtained with reference to exercise data collected by the wearable device by using a motion sensor, so as to detect a strike action of the user, and effectively assist the user in improving a strike rhythm.

According to a first aspect, this application provides a swing action detection method. The method is applied to a wearable device, and the wearable device includes one or more motion sensors and a sound collector. The wearable device collects first exercise data by using the one or more motion sensors, where the first exercise data includes acceleration data and angular velocity data. The wearable device collects a first sound signal by using the sound collector. The wearable device determines, when the wearable device determines that the first sound signal meets a first condition, that a user action is a strike action. When the wearable device determines that the first exercise data meets a second condition, the wearable device determines that a type of the strike action is a first strike action type. The wearable device displays the first strike action type.

The method implements detection on a strike action of a user, and helps improve stability of a strike rhythm of the user.

With reference to the first aspect, in a possible implementation of the first aspect, before the wearable device collects the first exercise data by using the one or more motion sensors, the wearable device displays a first user interface, where the first user interface displays a first control; the wearable device detects a first input performed on the first control; and in response to the first input, the wearable device collects the first exercise data by using the one or more motion sensors.

After the wearable device receives and responds to an input of the user, the wearable device further detects whether the one or more motion sensors and the sound collector are turned on. If the one or more motion sensors and the sound collector are not turned on, the wearable device turns on the one or more motion sensors and the sound collector. In this way, the one or more motion sensors and the sound collector are not in an always-on state, so that power consumption of the wearable device can be reduced.

With reference to the first aspect, in a possible implementation of the first aspect, before the wearable device collects the first exercise data by using the one or more motion sensors, the wearable device may further receive a first instruction sent by an electronic device in response to a second input, and in response to the first instruction, the wearable device collects the first exercise data by using the one or more motion sensors.

In this way, the wearable device establishes a communication connection to the electronic device, and then the electronic device may control the wearable device to collect the first exercise data and the first sound signal.

With reference to the first aspect, in a possible implementation of the first aspect, the wearable device determines, based on the first exercise data, an exercise parameter corresponding to the first strike action type, where the exercise parameter includes one or more of the following: an up-swing time, a down-swing time, a swing rhythm, and a strike speed, and the swing rhythm is a ratio of the up-swing time to the down-swing time.

The wearable device displays a second user interface, where the second user interface includes the exercise parameter corresponding to the first strike action type.

In this way, the wearable device may display an exercise parameter of the user to the user for viewing, so that the user knows an exercise status of the user. Therefore, the user adjusts an exercise posture of the user based on the exercise parameter, so that the exercise parameter reaches a standard value.

With reference to the first aspect, in a possible implementation of the first aspect, the wearable device determines, based on the first exercise data, an exercise parameter corresponding to the first strike action type, where the exercise parameter includes one or more of the following: an up-swing time, a down-swing time, a swing rhythm, a strike speed, and a quantity of strikes, and the swing rhythm is a ratio of the up-swing time to the down-swing time.

The wearable device sends, to the electronic device, an exercise parameter corresponding to the first strike action type, where the exercise parameter corresponding to the first strike action type may be displayed on a third user interface displayed on the electronic device.

Because an area of a display of the wearable device is limited, exercise parameters of the user that are displayed on the wearable device are limited. The wearable device may send exercise parameters of the user to the electronic device. The electronic device may display all exercise parameters of the user. The user can understand an exercise status of the user more comprehensively. Therefore, the user adjusts an exercise posture of the user based on the exercise parameters, so that the exercise parameters reach a standard value.

With reference to the first aspect, in a possible implementation of the first aspect, the user action includes any one of the following: a strike action, a whiff action, and a ground hit action.

With reference to the first aspect, in a possible implementation of the first aspect, that the wearable device determines, when the wearable device determines that the first sound signal meets a first condition, that a user action is a strike action specifically includes: The wearable device calculates a first feature parameter of the first sound signal, where the first feature parameter includes one or more of the following: an amount of energy, a frequency, and a peak value. The wearable device determines a first similarity based on the first feature parameter by using a first Gaussian mixture model, where the first similarity is used to indicate a similarity between the first feature parameter and a second feature parameter in the first Gaussian mixture model, and the second feature parameter includes one or more of the following: an amount of energy, a frequency, and a peak value. When the first similarity is greater than a first threshold, the wearable device determines that the user action is a strike action, where the first condition includes that the first similarity is greater than the first threshold.

With reference to the first aspect, in a possible implementation of the first aspect, that the wearable device determines, when the wearable device determines that the first sound signal meets the first condition and the first exercise data meets a third condition, that the user action is a strike action specifically includes: The wearable device calculates a first feature parameter of the first sound signal, where the first feature parameter includes one or more of the following: an amount of energy, a frequency, and a peak value. The wearable device determines a first similarity based on the first feature parameter by using a first Gaussian mixture model, where the first similarity is used to indicate a similarity between the first feature parameter and a second feature parameter in the first Gaussian mixture model, and the second feature parameter includes one or more of the following: an amount of energy, a frequency, and a peak value. The wearable device determines an acceleration waveform feature based on the first exercise data. When the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is greater than a first time threshold, the wearable device determines that the user action is a strike. The first condition includes that the first similarity is greater than the first threshold. The third condition includes that the quantity of peaks or troughs in the acceleration waveform feature is greater than the first quantity threshold, the maximum peak value is greater than the first peak threshold, the difference between the maximum peak value and the minimum trough value adjacent to the maximum peak value is greater than the first difference threshold, and the time in which the maximum peak value decreases to the first acceleration threshold is greater than the first time threshold.

At a down-swing end point, the user operates a club head of a golf club to prepare for striking a ball, but the club head does not hit the ball due to a mistake. However, in this case, a speed of the club head is quite high. If the wrist of the user shakes or the wearable device worn by the user is loose, the wearable device may also determine, through analysis based on the quantity of peaks and a peak value in the acceleration waveform feature, that the user is performing a strike action. Based on the foregoing impact, the wearable device may mistake a whiff action of the user as a strike action.

To eliminate an error, the wearable device collects a sound signal, performs similarity matching calculation on a feature parameter of a sound signal at a first moment and a Gaussian mixture model, and determines that the sound signal is generated by a strike. This eliminates misjudgment.

When the user operates the club head to hit the ball, the club head collides with the ball, and a loud sound is generated. A sound signal collected by the wearable device when the user performs a strike action is greatly different from a sound signal collected by the wearable device when the user performs a whiff action. In a spectrum graph of sound signals collected by the wearable device, a peak value and an amount of energy of a sound signal generated by a strike are far greater than a peak value and an amount of energy of a sound signal generated by a whiff. Based on this difference, the wearable device can distinguish whether the user is performing a strike action or a whiff action.

Therefore, the wearable device determines, with reference to the first condition and the second condition, that the user action is a strike action, so that a result is more accurate.

With reference to the first aspect, in a possible implementation of the first aspect, the wearable device determines, based on the first exercise data, that the user action is a strike action. That the wearable device determines, based on the first exercise data, that the user action is a strike action specifically includes: The wearable device determines an acceleration waveform feature based on the first exercise data. When the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is greater than a first time threshold, the wearable device determines that the user action is a strike.

The first condition includes that the quantity of peaks or troughs in the acceleration waveform feature is greater than the first quantity threshold, the maximum peak value is greater than the first peak threshold, the difference between the maximum peak value and the minimum trough value adjacent to the maximum peak value is greater than the first difference threshold, and the time in which the maximum peak value decreases to the first acceleration threshold is greater than the first time threshold.

With reference to the first aspect, in a possible implementation of the first aspect, the wearable device determines, based on the first exercise data, that the user action is a whiff action. That the wearable device determines, based on the first exercise data, that the user action is a whiff action specifically includes: The wearable device determines an acceleration waveform feature based on the first exercise data. When the wearable device determines that a quantity of peaks or troughs in the acceleration waveform feature is less than a first quantity threshold, a maximum peak value is less than a first peak threshold, a difference between the maximum peak value and a minimum trough value adjacent to the maximum peak value is less than a first difference threshold, and a time in which the maximum peak value decreases to a first acceleration threshold is greater than a first time threshold, the wearable device determines that the user action is a whiff.

With reference to the first aspect, in a possible implementation of the first aspect, the wearable device determines, based on the first exercise data, that the user action is a ground hit action. That the wearable device determines, based on the first exercise data, that the user action is a ground hit action specifically includes:

The wearable device determines an acceleration waveform feature based on the first exercise data. When the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is less than a first time threshold, the wearable device determines that the user action is a ground hit.

With reference to the first aspect, in a possible implementation of the first aspect, the first strike action type is a half-swing strike, and before the wearable device determines that the type of the strike action is the first strike action type, the method further includes: The wearable device determines a first rotation angle and/or a first moving distance of the wearable device within a first time based on the first exercise data, where the second condition is that the first rotation angle is less than or equal to a first preset angle and/or the first moving distance is less than or equal to a first preset distance within the first time.

With reference to the first aspect, in a possible implementation of the first aspect, the first strike action type is a full-swing strike, and before the wearable device determines that the type of the strike action is the first strike action type, the method further includes: The wearable device determines a first rotation angle and/or a first moving distance of the wearable device within a first time based on the first exercise data, where the second condition is that the first rotation angle is greater than a first preset angle and/or the first moving distance is greater than a first preset distance within the first time.

With reference to the first aspect, in a possible implementation of the first aspect, the wearable device counts a quantity of strikes within a second time period, and the wearable device counts a quantity of whiffs and a quantity of ground hits within the second time period.

The wearable device determines a strike rate based on the quantity of strikes within the second time period and the quantity of whiffs and the quantity of ground hits within the second time period, and the wearable device displays a fourth user interface, where the fourth user interface includes the strike rate.

In this way, the wearable device displays the strike rate to the user for viewing, so that the user can learn of a level of the user. In this way, the user can learn of an exercise status of the user, to better adjust a training plan of the user.

According to a second aspect, this application provides a wearable device, including one or more processors, one or more memories, one or more motion sensors, a sound collector, and a display. The one or more memories, the one or more motion sensors, the sound collector, and the display are coupled to the one or more processors. The one or more memories are configured to store computer program code. The computer program code includes computer instructions. The one or more processors invoke the computer instructions, so that the wearable device performs the swing action detection method provided in any implementation of the first aspect.

According to a third aspect, an embodiment of this application provides a computer storage medium. The computer-readable storage medium stores a computer program. When the computer program is executed by a processor, the processor performs the swing action detection method provided in any implementation of the first aspect.

According to a fourth aspect, an embodiment of this application provides a computer program product. A computer-readable storage medium stores a computer program. When the computer program is executed by a processor, the processor performs the swing action detection method provided in any implementation of the first aspect.

In the method, the wearable device can collect a sound signal by using the sound collector, to determine whether a user strikes a ball. In addition, a swing action parameter of the user is obtained with reference to exercise data collected by the wearable device by using a motion sensor, so as to detect a strike action of the user, and effectively assist the user in improving a strike rhythm.

## BRIEF DESCRIPTION OF DRAWINGS

FIG. 1 is a schematic diagram of a structure of a wearable device 100 according to an embodiment of this application;
FIG. 2 to FIG. 7 are schematic diagrams of a group of golf actions according to an embodiment of this application;
FIG. 8 and FIG. 9 are diagrams of a group of UIs according to an embodiment of this application;
FIG. 10 is a diagram of a UI of a wearable device 100 according to an embodiment of this application;
FIG. 11 to FIG. 13 are diagrams of UIs on which an electronic device 200 sends an instruction for enabling a golf mode to a wearable device 100 according to an embodiment of this application;
FIG. 14 to FIG. 19 are diagrams of another group of UIs according to an embodiment of this application;
FIG. 20 to FIG. 22 are diagrams of another group of UIs according to an embodiment of this application;
FIG. 23 is a flowchart of a golf action detection method according to an embodiment of this application;
FIG. 24A is a schematic diagram of an acceleration waveform feature and an angular velocity waveform feature in the case of a strike according to an embodiment of this application;
FIG. 24B is a schematic diagram of an acceleration waveform feature and an angular velocity waveform feature in the case of a whiff according to an embodiment of this application;
FIG. 24C is a schematic diagram of an acceleration waveform feature and an angular velocity waveform feature in the case of a ground hit according to an embodiment of this application;
FIG. 25 and FIG. 26 are diagrams of a group of LJIs on which a wearable devices 100 displays exercise data according to an embodiment of this application;
FIG. 27 to FIG. 29 are diagrams of a group of UIs on which an electronic devices 200 displays exercise data according to an embodiment of this application;
FIG. 30 is a diagram of a hardware structure of an electronic device 200 according to an embodiment of this application; and
FIG. 31 is a schematic diagram of a structure of a wearable device 100 according to an embodiment of this application.

## DESCRIPTION OF EMBODIMENTS

The following clearly describes technical solutions in embodiments of this application in detail with reference to accompanying drawings. In descriptions of embodiments of this application, "/" indicates "or", unless otherwise specified. For example, A/B may indicate A or B. The term "or" in this specification describes only an association relationship for describing associated objects, and indicates that three relationships may exist. For example, A or B may indicate the following three cases: Only A exists, both A and B exist, and only B exists. In addition, in descriptions of embodiments of this application, "a plurality of" means two or more than two.

Terms "first" and "second" mentioned below are merely intended for a purpose of description, and shall not be understood as an implication or implication of relative importance or an implicit indication of a quantity of indicated technical features. Therefore, a feature limited by "first" or "second" may explicitly or implicitly include one or more features. In descriptions of embodiments of this application, "a plurality of" means two or more than two, unless otherwise specified.

A term "user interface (user interface, UI)" in this specification, the claims, and the accompanying drawings of this application is a medium interface for interaction and information exchange between a user and an application or an operating system, and the interface implements conversion between an internal form of information and a form that can be accepted by the user. A user interface of an application is source code written by using a specific computer language such as Java or extensible markup language (extensible markup language, XML). The interface source code is parsed and rendered on a terminal device, and finally is presented as content that can be identified by a user, for example, a control such as a picture, a text, or a button. The control (control), also referred to as a widget (widget), is a basic element on the user interface. Typical controls include a toolbar (toolbar), a menu bar (menu bar), a text box (text box), a button (button), a scrollbar (scrollbar), a picture, and a text. An attribute and content of a control on an interface are defined by using a tag or a node. For example, the control included in the interface is defined in the XML by using a node, for example, <Textview>, <ImgView>, or <VideoView>. One node corresponds to one control or one attribute on the interface. After being parsed and rendered, the node is presented as user-visible content. In addition, interfaces of many applications such as a hybrid application (hybrid application) usually further include a web page. A web page, also referred to as a page, may be understood as a special control embedded in an application interface. A web page is source code written by using a specific computer language such as hypertext markup language (hypertext markup language, HTML), cascading style sheets (cascading style sheets, CSS), or JavaScript (JavaScript, JS). The web page source code may be loaded and displayed, as content that can be identified by a user, by a browser or a web page display component with a function similar to that of the browser. Specific content included in the web page is also defined by using a tag or a node in the web page source code. For example, an element and an attribute of the web page are defined in the HTML by using <p>, <img>, <video>, or <canvas>.

The user interface is usually represented in a form of a graphical user interface (graphical user interface, GUI), and is a user interface that is related to a computer operation and that is displayed in a graphic manner. The user interface may be an interface element such as an icon, a window, or a control displayed on a display of an electronic device, and the control may include a visual interface element such as an icon, a button, a menu, a tab, a text box, a dialog box, a status bar, a navigation bar, or a widget.

An embodiment of this application provides a swing action detection method. A wearable device may identify, by using data detected by one or more motion sensors (for example, an acceleration sensor, a gyro sensor, and a sound signal collector), various ball-and-stick sports actions (for example, a strike action and a whiff action) performed by a user, and detect a standardization degree of completing the ball-and-stick sports actions by the user. This can help the user correct the ball-and-stick sports action. Ball-and-stick sports may be sports such as hockey, badminton, billiards, baseball, golf, and tennis.

In embodiments of this application, golf sports is specifically used as an example for description. However, this should not be construed as a limitation.

Specifically, after collecting acceleration data and angular velocity data of the wrist of the user by using the motion sensors (the acceleration sensor and the gyro sensor), the wearable device (for example, a Bluetooth band or a Bluetooth watch) may further collect a sound signal by using the sound signal collector. The wearable device may obtain an acceleration waveform feature and an angular velocity waveform feature based on the acceleration data and the angular velocity data respectively. The wearable device may determine, through analysis based on a quantity of peaks/troughs, a peak value, and a peak-trough difference in each of the acceleration waveform feature and the angular velocity waveform feature, that a user action is any one of a whiff, a ground hit, and a strike. When the user performs ball-and-stick sports, the sound signal collected by the sound collector may include a sound signal generated by a strike and a sound signal generated by a whiff. The wearable device may identify, by using a trained Gaussian mixture model, the sound signal collected by the sound collector, to distinguish between the sound signal generated by the strike and the sound signal generated by the whiff. When the wearable device determines, through analysis, that the user is performing a strike action, the wearable device may further determine, through analysis by using the acceleration data and the angular velocity data, a swing action parameter of the user, for example, data such as a swing rhythm. In this way, the wearable device may determine, through analysis based on the sound signal, whether the user is performing a strike or a whiff, and calculate the swing action parameter of the user based on the acceleration data and the angular velocity data. The method implements detection on a strike action of the user, and helps improve stability of a strike rhythm of the user.

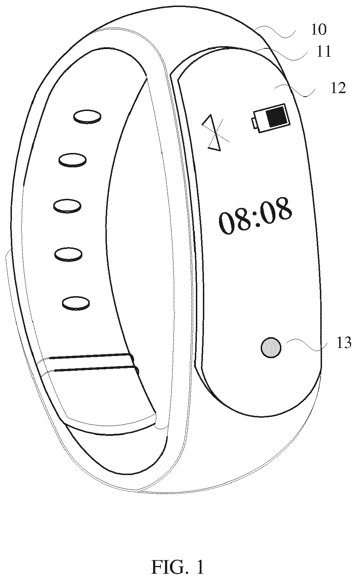

The following describes a terminal, namely, a wearable device, in embodiments of this application. FIG. 1 is a schematic diagram of a structure of a wearable device 100. The wearable device 100 may include a device body 11 and a wearable part 10.

The device body 11 is equipped with an acceleration sensor, a gyro sensor, and a sound collector. The device body 11 may collect acceleration data, angular velocity data, and a sound signal by using the acceleration sensor, the gyro sensor, and the sound collector respectively. The device body 11 may include a display 12 and a touch control 13. The display 12 may be configured to display content such as a time, a battery level of the device body 11, a Bluetooth identifier, a received message, and exercise data of a user. The touch control 13 may be configured to receive a tap operation of the user to turn on the display, enable or disable a sports mode, or the like.

The device body 11 may further record a quantity of moving steps and an amount of consumed energy of the user, and has basic functions such as an incoming call reminder and a message notification.

The device body 11 may establish a wireless communication connection to an electronic device 200 (for example, a mobile phone or a tablet computer).

In a possible implementation, the device body 11 may establish a wireless communication connection to the electronic device 200 through Bluetooth. The device body 11 may send the exercise data of the user to the electronic device 200 to which the connection is established. In addition, when the electronic device 200 receives an incoming call or message notification, the device body 11 may receive an instruction from the mobile phone, to inform the user of the incoming call or message notification.

The wearable part 10 is configured to install the device body 11. For example, the wearable part 10 may be an apparatus such as a band strap or a watch strap. The wearable part 10 is an apparatus that can attach the device body 11 to the wrist of the user. The wearable device 100 is attached to the wrist of the user. This helps the acceleration sensor and the gyro sensor collect acceleration data and angular velocity data of the wrist of the user, to detect movement of the wrist of the user, and also helps the sound collector collect a sound signal generated by a club head and a ball when the user performs a strike action, to detect whether the user performs a strike.

For ease of understanding this application, the following describes, by using a golf application scenario, a swing action detection method provided in embodiments of this application.

First, terms involved in the golf application scenario are explained.

Golf mode: The golf mode is a function of a wearable device 100, and may be used to record exercise data of a user when the user plays golf, and detect an action of playing golf by the user. When the golf mode is enabled, an acceleration sensor, a gyro sensor, and a sound collector in the wearable device 100 each are in an operating state, and may be configured to collect acceleration data, angular velocity data, and a sound signal of the wrist respectively when the user plays golf.

The wearable device 100 may process the acceleration data, the angular velocity data, and the sound signal to obtain exercise data such as an up-swing time, a down-swing time, and a swing rhythm, so as to determine whether the user performs a whiff action or a strike action when playing golf.

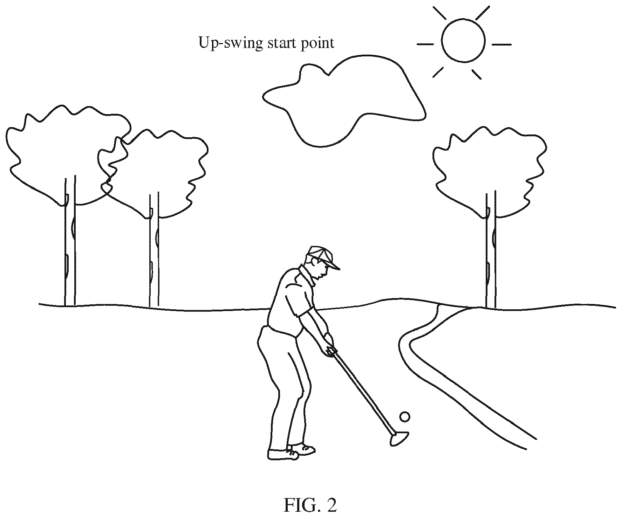

Up-swing start point: FIG. 2 is a schematic diagram in which the user is aiming a club head of a golf club at a ball. The up-swing start point is a point at which the user aims the club head of the golf club at the ball, and makes the golf club perpendicular to a horizontal ground and remain stationary. At the up-swing start point, a moving distance of the wrist in a vertical direction is a minimum value. In this way, after finding the up-swing start point, the user may prepare for a subsequent strike, so that strike accuracy of the user can be improved.

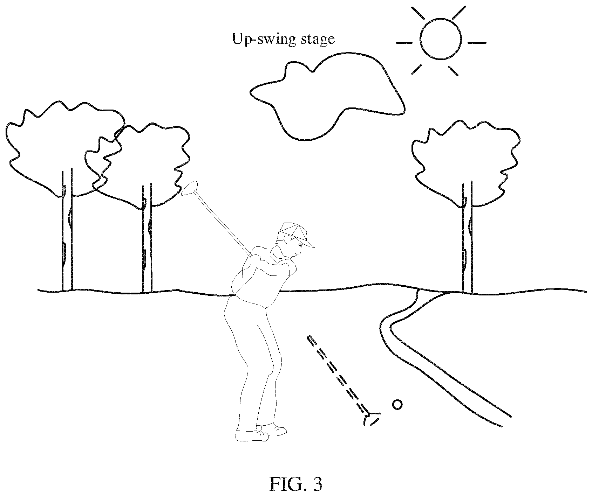

Up-swing stage: FIG. 3 is a schematic diagram of the up-swing stage. The user tightly holds the golf club with both hands. Starting from the up-swing start point, the user rotates the shoulders to rotate the golf club toward the upper right (or upper left), until the wrist of the user rises to a highest point above the horizontal ground. An action that the user operates the golf club from the up-swing start point until the wrist of the user rises to the highest point above the horizontal ground is referred to as an up swing. In the up-swing stage, a process in which the moving distance of the wrist in the vertical direction changes from the minimum value to a maximum value is referred to as the up-swing stage.

Up-swing end point: After the user ends the up-swing action, a location at which the wrist of the user stays is the up-swing end point. At the up-swing end point, a distance to which the wrist of the user rises from the horizontal ground is a highest location above the horizontal ground in the up-swing stage. At the up-swing end point, the moving distance of the wrist in the vertical direction is the maximum value.

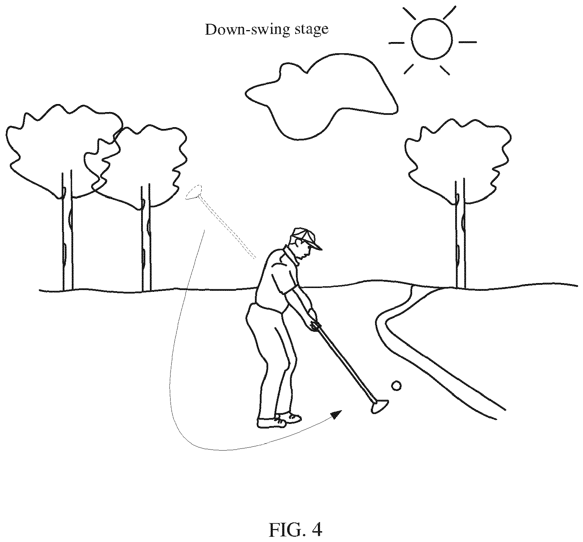

Down swing stage: FIG. 4 is a schematic diagram of the down-swing stage. The user tightly holds the golf club with both hands. Starting from the up-swing end point, the user rotates the shoulders to start to rotate the golf club toward the lower left (or lower right), until the club head of the golf club rises to a lowest point above the horizontal ground. An action that the user operates the golf club from the up-swing end point until the club head reaches the lowest point above the horizontal ground is referred to as a down swing. In the down-swing stage, a process in which the moving distance of the wrist in the vertical direction changes from the maximum value to the minimum value is referred to as the down-swing stage.

Down swing end point: After the user ends the down-swing action, a location at which the club head of the golf club stays is the down-swing end point. At the down-swing end point, a distance from the club head of the golf club to the horizontal ground is a location at the lowest point above the horizontal ground in the down-swing stage. In addition, the down-swing end point is close to a strike point. At the down-swing end point, the moving distance of the wrist in the vertical direction is the minimum value.

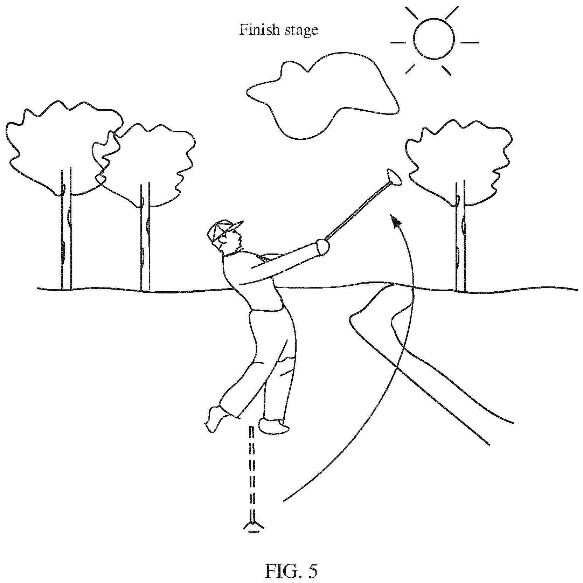

Finish stage: FIG. 5 is a schematic diagram of the finish stage. After the user operates the club head of the golf club to perform a strike or a whiff, due to inertia, the golf club still moves forward by a specific distance until the golf club stops. In the finish stage, a process in which an absolute value of a displacement of the wrist in the vertical direction changes from zero to the maximum value again is referred to as the finish stage.

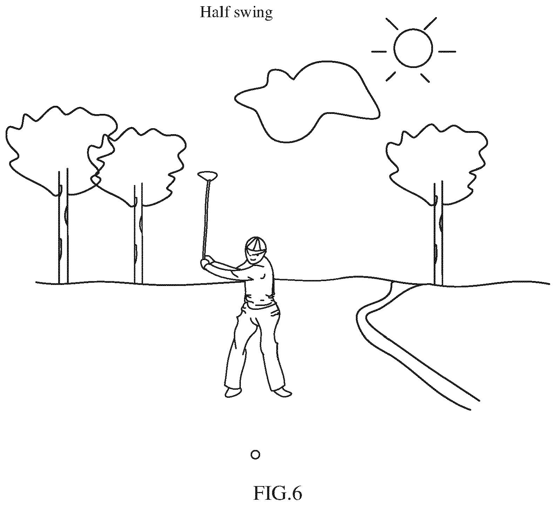

Half swing: FIG. 6 is a schematic diagram of an up-swing stage in a half swing in a golf action. The half swing includes an up-swing stage, a down-swing stage, and a finish stage. During the up-swing stage, whether a half swing is being performed may be determined based on a rotation angle of the wrist and/or a moving distance of the wrist in the vertical direction.

Manner 1: The wearable device 100 may determine, based on the rotation angle of the wrist, that a half swing is being performed. Specifically, within an up-swing time, if the rotation angle Q of the wrist is less than or equal to a first preset angle (for example, 90 degrees), the wearable device 100 determines that a half swing is being performed.

Manner 2: The wearable device 100 determines, based on the moving distance of the wrist in the vertical direction, that a half swing is being performed. Specifically, within an up-swing time, if the moving distance S of the wrist in the vertical direction is less than or equal to a first preset distance, the wearable device 100 determines that a half swing is being performed. Usually, when the user performs a half swing action, arms of the user are parallel to the ground, and an average value of vertical distances between shoulders of a plurality of users and the ground may be used as the first preset distance. Alternatively, the first preset distance may be set in another manner. This is not limited in this application.

Manner 3: The wearable device 100 determines, based on the rotation angle of the wrist and the moving distance of the wrist in the vertical direction, that a half swing is being performed. Specifically, within an up-swing time, if the rotation angle Q of the wrist is less than or equal to a first preset angle (for example, 90 degrees) and the moving distance S of the wrist in the vertical direction is less than or equal to a first preset distance, the wearable device 100 determines that a half swing is being performed.

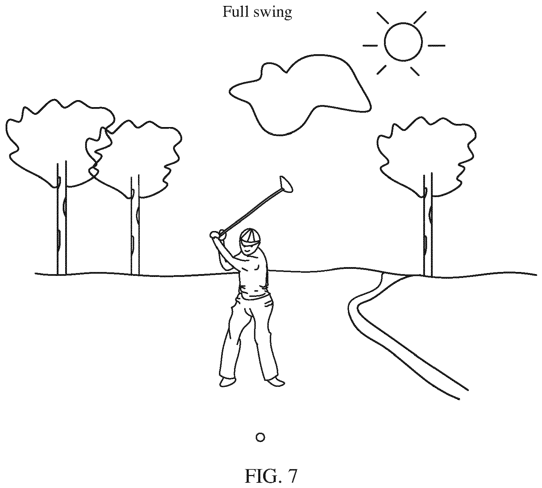

Full swing: FIG. 7 is a schematic diagram of a full swing in a golf action. The full swing includes an up-swing stage, a down-swing stage, and a finish stage. During the up-swing stage, whether a full swing is being performed may be determined based on a rotation angle of the wrist and/or a moving distance of the wrist in the vertical direction.

Manner 1: The wearable device 100 determines, based on the rotation angle of the wrist, that a full swing is being performed.

Specifically, within an up-swing time, if the rotation angle Q of the wrist is greater than a second preset angle (for example, 180 degrees), the wearable device 100 determines that a full swing is being performed.

Manner 2: The wearable device 100 determines, based on the moving distance of the wrist in the vertical direction, that a full swing is being performed.

Specifically, within an up-swing time, if the moving distance S of the wrist in the vertical direction is greater than a second preset distance, the wearable device 100 determines that a full swing is being performed.

Manner 3: The wearable device 100 determines, based on the rotation angle of the wrist and the moving distance of the wrist in the vertical direction, that a full swing is being performed.

Specifically, within an up-swing time, if the rotation angle Q of the wrist is greater than a second preset angle (for example, 180 degrees) and the displacement S of the wrist in the vertical direction is greater than a second preset distance, the wearable device 100 determines that a full swing is being performed.

The first preset angle is less than the second preset angle, and the first preset distance is less than the second preset distance.

Up-swing time: A time in which the user controls the golf club from the up-swing start point to the up-swing end point is the up-swing time.

Down-swing time: A time in which the user controls the golf club from the up-swing end point to the down-swing end point is the down-swing time.

Swing rhythm: A ratio of the up-swing time to the down-swing time is the swing rhythm.

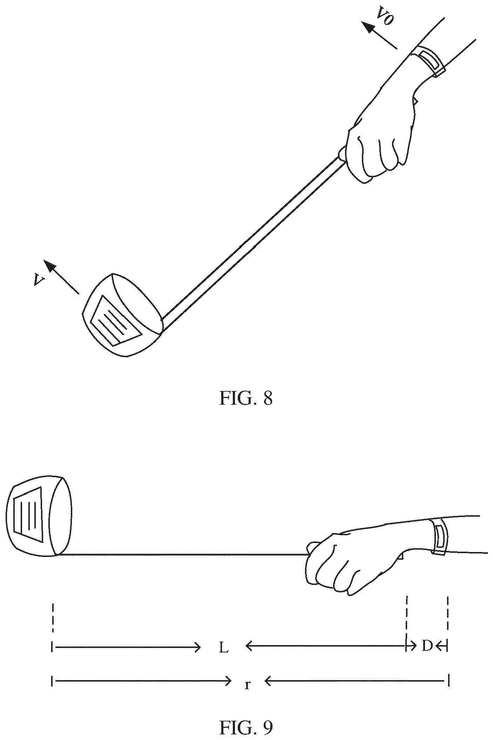

Swing speed: In the following embodiments of this application, the swing speed is an actual rotational speed of a club head of a golf club. FIG. 8 is a schematic diagram of a rotational speed of the club head of the golf club. The wearable device 100 collects acceleration data and angular velocity data of the wrist of the user. The wearable device 100 may calculate a rotational speed V0 of the wrist of the user based on the collected acceleration data and angular velocity data, and then calculate an actual rotational speed V of the club head of the golf club based on a formula.

A relative speed of the club head is $V1 = w \times r$, and the actual rotational speed of the club head is $V = V1 + V0$, where w is a real-time rotational angular velocity of the wrist, r is a rotation radius of the club head of the golf club, that is, a distance from the wrist wearing the wearable device 100 to the club head of the golf club, w is an angular velocity vector, and V, V0, and V1 are all speed vectors.

To calculate the actual rotational speed V of the club head of the golf club, the rotation radius r of the club head of the golf club needs to be estimated.

Usually, a posture of holding the golf club by the user with both hands is that the left hand is above the right hand. Therefore, the wearable device 100 needs to identify whether the wearable device 100 is worn on the left hand or the right hand, and then estimate the rotation radius r of the club head of the golf club based on a left-handed or right-handed wearing compensation coefficient and a preset club length of the golf club. The left-handed or right-handed wearing compensation coefficient is a distance from the wrist of the left hand or the wrist of the right hand to a club handle of the golf club. The left-handed or right-handed wearing compensation coefficient is preset on the wearable device 100. In this way, the left-handed or right-handed wearing compensation coefficient is added during the calculation of the rotation radius r of the club head of the golf club, so that an error of calculating the actual rotational speed of the club head of the golf club can be reduced.

Left-handed wearing:

FIG. 9 is a schematic diagram in which the wearable device 100 is worn on the wrist of the left hand of the user.

The wearable device 100 may automatically identify whether the wearable device 100 is worn on the left hand or the right hand of the user. When identifying that the wearable device 100 is worn on the wrist of the left hand of the user, the wearable device 100 estimates the rotation radius r of the club head of the golf club based on a preset distance D (for example, 0.1 meters) from the wrist of the left hand to the club handle of the golf club and a club length L (for example, 0.9 meters) of the golf club, where r = D + L.

When identifying that the wearable device 100 is worn on the wrist of the right hand of the user, the wearable device 100 may determine the rotation radius r of the club head of the golf club based on a preset distance D (for example, 0.1 meters) from the wrist of the right hand to the club handle of the golf club and a club length L (for example, 0.9 meters) of the golf club, where r = D + L.

When the wearable device 100 enables the golf mode, the wearable device 100 may determine that collected exercise data comes from golf sports rather than other sports such as running. The wearable device 100 may receive an input of the user and enables the golf mode; or determine, based on data collected by a sensor, to enable the golf mode.

Manner 1: The wearable device 100 receives and responds to the input of the user, and enables the golf mode.

In some embodiments, the wearable device 100 may enable the golf mode by detecting a user operation performed on the touch control 13.

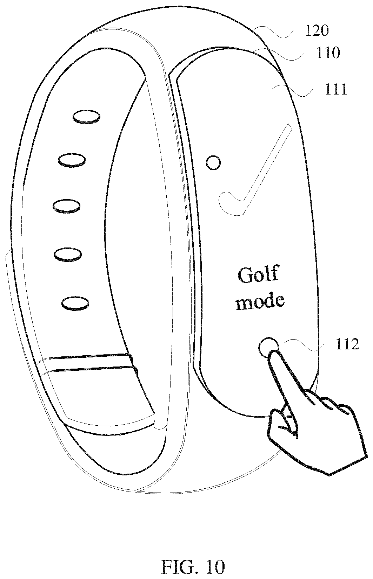

For example, as shown in FIG. 10, when the wearable device 100 detects a touch-and-hold operation performed on the touch control 13, the wearable device 100 may enable or disable the golf mode. When the wearable device 100 has not enabled the golf mode and detects a touch-and-hold operation performed on the touch control 13, the wearable device 100 may count down for a specific time (for example, 3 seconds) after vibration, and enable the golf mode. After the golf mode is enabled, the wearable device 100 may display a golf icon and texts "golf mode" on the display 12. In this way, the user may be notified that the wearable device 100 has enabled the golf mode.

When the wearable device 100 has enabled the golf mode and detects a touch-and-hold operation performed on the touch control 13, the wearable device 100 may count down for a specific time (for example, 3 seconds) after vibration, and disable the golf mode.

In a possible implementation, the wearable device 100 may automatically turn off the display 12 after the golf mode is enabled for a period of time (for example, 1 minute or 2 minutes). When detecting a touch operation performed on the touch control 13, the wearable device 100 may turn on the display 12. After being turned on, the display 111 may display the golf icon and the texts "golf mode" shown in FIG. 10. Further, if detecting a touch-and-hold operation performed on the touch control 13, the wearable device 100 may count down for a specific time (for example, 3 seconds) after vibration, and disable the golf mode.

In a possible implementation, the wearable device 100 automatically turns off the display 12 after the golf mode is enabled for a period of time, so that power consumption of the wearable device 100 can be effectively reduced.

In some other embodiments, after the wearable device 100 establishes a communication connection to the electronic device 200, the wearable device 100 may alternatively enable the golf mode by receiving a golf mode enabling request sent by the electronic device 200.

The wearable device 100 may establish a communication connection relationship with the electronic device 200 (for example, a mobile phone or a tablet computer). When detecting a user operation used to enable or disable the golf mode, the electronic device 200 may send, to the wearable device 100, an instruction for enabling the golf mode. When the wearable device 100 receives the instruction used to enable the golf mode, the wearable device 100 may enable the golf mode.

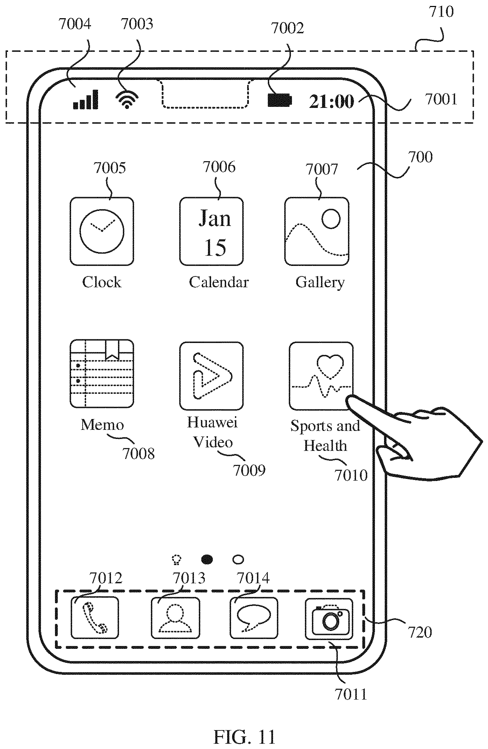

As shown in FIG. 11, the electronic device 200 displays a home screen user interface 700. The user interface 700 may include a status bar 710, a tray 720 with common application icons, and other application icons. The status bar 710 may include a time indicator 7001, a battery status indicator 7002, one or more signal strength indicators 7003 for a wireless fidelity (wireless fidelity, Wi-Fi) signal, and one or more signal strength indicators 7004 for a mobile communication signal (which may also be referred to as a cellular signal). The tray 720 with common application icons may display a "Camera" icon 7011, a "Phone" icon 7012, a "Contacts" icon 7013, and a "Messages" icon 7014. The other application icons may be, for example, a "Clock" icon 7005, a "Calendar" icon 7006, a "Gallery" icon 7007, a "Memo" icon 7008, a "Huawei Video" icon 7009, or a "Sports and Health" icon 7010, an icon 7010 of a "Sports and Health" application, and an icon of another application (for example, "Gallery"). An icon of any application may be configured to respond to a user operation (for example, a tap operation), so that the electronic device 200 starts an application corresponding to the icon. The "Sports and Health" icon 7010 may be configured to start a "Sports and Health" application. The "Sports and Health" application may be used by the electronic device 200 to establish a communication connection relationship with the wearable device 100. The electronic device 200 may display exercise data of a user to the user by using the "Sports and Health" application.

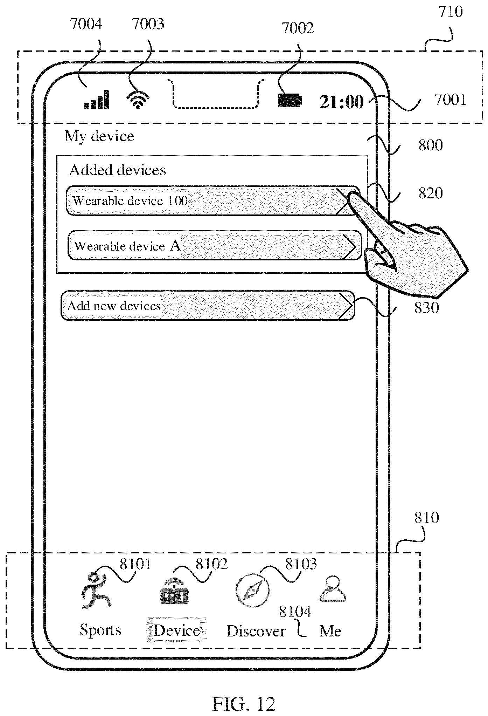

The electronic device 200 receives and responds to a user operation (for example, a tap operation) performed on the "Sports and Health" icon 7010, and the electronic device 200 may display a "Sports and Health" application interface 800 shown in FIG. 12.

As shown in FIG. 12, the application interface 800 may include a status bar 710 and an interface view option 810. The interface view option 810 may include a "Sports" option 8101, a "Device" option 8102, a "Discover" option 8103, and a "Me" option 8104. Any option may be configured to respond to a user operation (for example, a tap operation), so that the electronic device 200 displays, on the application interface 800, content corresponding to the option. For example, content corresponding to the device option 8102 may include device information that has been added to the electronic device and a control configured to add a new device. When the electronic device 200 detects a user operation (for example, a tap operation) performed on the device option 8102, the electronic device 200 may display an "Added devices" option 820 and a device adding option 830.

The device adding option 830 may be configured to trigger the electronic device 200 to add a new device. The new device is a device that establishes a communication connection relationship with the electronic device 200 for the first time. When the electronic device 200 detects a user operation (for example, a tap operation) performed on the device adding option 830, the electronic device 200 may display a device adding settings interface, so that the electronic device 200 establishes a communication connection relationship with the new device. The device adding settings interface may be used for the user to search for a new device, a mode for establishing a communication connection, for example, a Bluetooth connection, or the like. A process of establishing a communication connection relationship between the electronic device 200 and the new device is not limited in this embodiment of this application.

The "Added devices" option 820 may include identifiers of a plurality of wearable devices. All the plurality of wearable devices have established communication connection relationships with the electronic device 200. For example, the electronic device 200 has established communication connection relationships with the wearable device 100 and a wearable device A. When detecting a user operation (for example, a tap operation) performed on any device option in the "Added devices" option 820, the electronic device may display related information corresponding to the device.

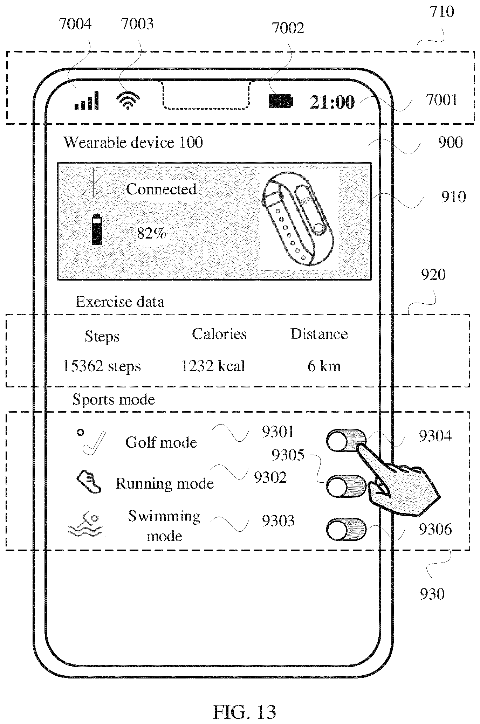

When the electronic device 200 detects a user operation (for example, a tap operation) performed on an identifier of the wearable device 100 in the "Added devices" option 212, the electronic device 200 may display an application interface 900 shown in FIG. 13.

As shown in FIG. 13, the application interface 900 may include a status bar 710, a device status bar 910, exercise data 920, and a sports mode option 930. The device status bar 910 may be configured to display a connection status between the wearable device 100 and the electronic device 200 and a battery level of the wearable device 100. For example, when it is detected that the electronic device 200 establishes a communication connection relationship with the wearable device 100 in a Bluetooth connection mode, the device status bar 910 may prompt that a connection mode is a Bluetooth connection and a connection status is "connected". Further, the electronic device 200 may obtain battery level information of the wearable device 100. The device status bar 910 may prompt a current battery level (for example, 77%) of the wearable device 100. Content prompted in the device status bar 910 may further include more content. This is not limited in this embodiment of this application. The exercise data 920 may include a quantity of moving steps of the user, an amount of consumed energy, and a moving distance that are recorded by the wearable device 100. Data in the exercise data 920 is data, recorded by the wearable device 100 in an operating state, of the user in one day (for example, including a total quantity of moving steps, a total amount of consumed energy, and a total moving distance of the user in activities such as daily walking, golf playing, and running).

The sports mode option 930 may be configured to enable or disable the golf mode, a running mode, and a swimming mode. The sports mode option 930 may include a golf mode identifier 9301, a control 9304 used to enable the golf mode, a running mode identifier 9302, a control 9305 used to enable the running mode, a swimming mode identifier 9303, and a control 9306 used to enable the swimming mode. In response to a user operation (for example, tapping) performed on the control 9304 for enabling the golf mode, the electronic device 200 may send, to the wearable device 100, an instruction for enabling the golf mode. When the wearable device 100 receives the instruction for enabling the golf mode that is sent by the electronic device 200, the wearable device 100 may enable the golf mode after a specific time (for example, a countdown of 3 seconds) after vibration. When the golf mode is enabled, the wearable device 100 may display the golf icon and the texts "golf mode" on the display 12. In this way, the user may be notified that the wearable device 100 has enabled the golf mode.

When receiving an instruction for disabling the golf mode that is sent by the electronic device 200, the wearable device 100 may disable the golf mode after a specific time (for example, a countdown of 3 seconds) after vibration.

In addition, when the golf mode is enabled, the wearable device 100 may automatically enable a do-not-disturb mode. For example, when the do-not-disturb mode is enabled and the electronic device 200 receives an incoming call or message notification, the wearable device 100 may mask a notification instruction, sent by the electronic device, for the incoming call or message notification. That is, the wearable device 100 does not notify, in a manner such as vibration or ringing, the user of the incoming call or message notification. In this way, when the wearable device 100 has enabled the golf mode and there is an incoming call or message notification, the wearable device 100 does not interfere with the user in playing golf.

Manner 2: The wearable device 100 determines, based on data collected by a sensor, to enable the golf mode.

Alternatively, the wearable device 100 may adaptively enable the golf mode.

When the wearable device 100 has not enabled the golf mode and detects that the user is in a state of playing golf, the wearable device 100 may automatically enable the golf mode. A manner of determining, by the wearable device 100, that the user is in the state of playing golf may be as follows: The acceleration sensor and the gyroscope sensor collect acceleration data and angular velocity data. The wearable device 100 obtains an acceleration waveform feature map and an angular velocity waveform feature map based on the acceleration data and the angular velocity data. The wearable device 100 may determine, through calculation based on the acceleration waveform feature map and the angular velocity waveform feature map, whether the user has a strike point. If the wearable device 100 determines, through calculation, that the user has a strike point, it is determined that the user is performing suspected golf sports. The wearable device 100 inputs the acceleration waveform feature map and the angular velocity waveform feature map to a classification model. The classification model is trained in advance. The classification model may analyze a sports type (for example, running, swimming, or playing golf) of the user based on input exercise data. The classification model determines, through analysis based on the acceleration waveform feature map and the angular velocity waveform feature map, that the user is playing golf. In this case, the wearable device 100 may determine that the user is in the state of playing golf.

Herein, after obtaining the acceleration waveform feature map and the angular velocity waveform feature map, the wearable device 100 determines that the user is performing suspected golf sports, and then inputs the acceleration waveform feature map and the angular velocity waveform feature map to the classification model. The classification model further determines that the user is playing golf. However, the acceleration data and the angular velocity data that are collected by the acceleration sensor and the gyro sensor are not directly input to the classification model. This can reduce an amount of computation performed by the classification model, reduce consumption, and improve accuracy of determining performed by the classification model.

In some other embodiments, alternatively, the wearable device 100 may directly input the acceleration data and the angular velocity data that are collected by the acceleration sensor and the gyroscope sensor to the classification model, and the classification model directly outputs that the user is playing golf or that the user is not playing golf.

Alternatively, the wearable device 100 may determine, in another manner, that the user is playing golf. This is not limited in this embodiment of this application.

In some embodiments, when the wearable device 100 has enabled another sports mode, for example, the running mode, but detects that the user is in a state of playing golf, the wearable device 100 may automatically switch from the another sports mode to the golf mode.

In some embodiments, when the wearable device 100 has enabled the golf mode and detects that the user is not in a state of playing golf, the wearable device 100 may automatically disable the golf mode. A manner of determining, by the wearable device 100, that the user is not in a state of playing golf may be as follows: The wearable device 100 determines, through calculation based on a sound signal, acceleration data, and angular velocity data that are collected, that a quantity of strike points is zero. In this case, the wearable device 100 may determine that the user is not in a state of playing golf. Alternatively, the wearable device 100 may determine, in another manner, that the wearable device 100 is not in a state of playing golf. This is not limited in this embodiment of this application.

In this way, when the user forgets to enable the golf mode while playing golf or when an incorrect sports mode is enabled, the wearable device 100 may adaptively enable the golf mode, and detect an overall action and exercise data of the user when the user plays golf this time, to assist the user in improving an ability to play golf. In addition, when the user forgets to disable the golf mode after enabling the golf mode, the wearable device 100 may adaptively disable the golf mode, to reduce power consumption of the wearable device 100.

In addition to the foregoing manners of enabling the golf mode by detecting a user operation performed on the touch control 13 and by receiving an instruction sent by the electronic device 200, and the foregoing manner of adaptively enabling the golf mode, the wearable device 100 may alternatively enable the golf mode in another manner, for example, enable the golf mode by using a motion sensing gesture. This is not limited in this embodiment of this application.

In some embodiments, before enabling the golf mode, the wearable device 100 may detect whether a motion sensor (for example, the acceleration sensor and the gyro sensor) and the sound collector are in an operating state.

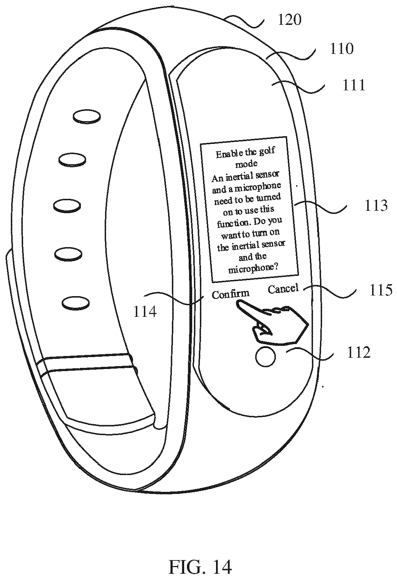

FIG. 14 is another schematic diagram of enabling the golf mode by the wearable device 100. When detecting a user operation (for example, a touch-and-hold operation performed on the touch control 13) used to enable the golf mode, the wearable device 100 may display, on the display 12, a user interface shown in FIG. 14. The user interface may include a prompt box 113, a "Confirm" control 14, and a "Cancel" control 115.

The prompt box 113 includes prompt content. The prompt content is used to prompt that the motion sensor (for example, the acceleration sensor and the gyro sensor) and the sound collector in the wearable device 100 are to be in an operating state when the golf mode is enabled, so that the user determines whether the user needs to enable the golf mode. The prompt content may include: "An inertial sensor and a microphone need to be turned on to use this function. Do you want to turn on the inertial sensor and the microphone?".

The "Confirm" control 14 may be configured to enable the golf mode. In response to a user operation (for example, a tap operation) performed on the golf, the wearable device 100 may detect whether the motion sensor (for example, the acceleration sensor and the gyro sensor) and the sound collector are in an operating state. If the motion sensor (for example, the acceleration sensor and the gyro sensor) and the sound collector are not in an operating state, the wearable device 100 may automatically enable the motion sensor (for example, the acceleration sensor and the gyro sensor) and the sound collector to be in an operating state. In this way, the wearable device 100 can enable the golf mode. In addition, the wearable device 100 vibrates for a period of time (for example, 3 seconds) after the golf mode is enabled, to notify the user that the golf mode is enabled.

The "Cancel" control 115 may be configured to cancel enabling the golf mode. In response to a user operation (for example, a tap operation) performed on the golf, the wearable device 100 does not enable the golf mode.

After the wearable device 100 enables the golf mode, the wearable device 100 may further prompt the user to select a time for inputting personal exercise data. In this way, the wearable device 100 can input exercise data of the user within a predetermined time.

The wearable device 100 may input exercise data of the user in either of the following two manners.

Manner 1: The wearable device 100 receives a user operation and inputs exercise data of the user.

Manner 2: The electronic device 1200 prompts the user to input exercise data.

First, that the wearable device 100 receives a user operation and inputs exercise data of the user is described.

When the wearable device 100 enables the golf mode for the first time, the wearable device 100 may prompt the user to input personal exercise data.

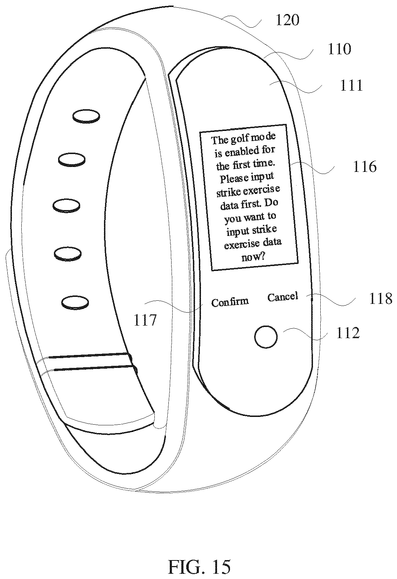

In some embodiments, after the wearable device 100 enables the golf mode, the wearable device 100 may display on the display 12, a user interface shown in FIG. 15. The user interface may include a prompt box 116, a "Confirm" control 117, and a "Cancel" control 118.

The prompt box 116 includes prompt content. The prompt content is used by the wearable device 100 to prompt the user to input exercise data after the golf mode is enabled. The prompt content may include: "The golf mode is enabled for the first time. Please input strike exercise data first. Do you want to input strike exercise data now?".

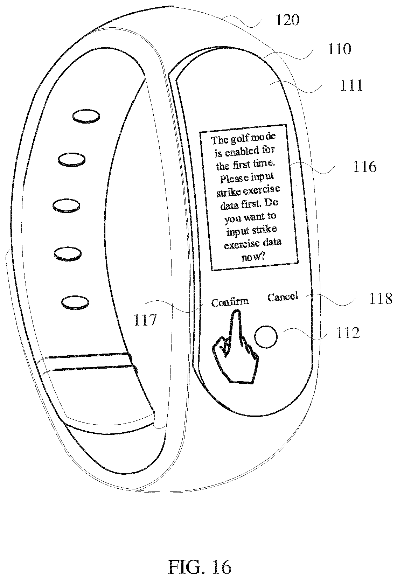

The "Confirm" control 117 may be configured to receive a user operation (for example, tapping). In response to the user operation (for example, tapping) performed on a "Confirm" option 311, the wearable device 100 may display on the display 12, a user interface shown in FIG. 16.

The "Cancel" control 118 may be configured to cancel inputting personal exercise data. In response to a user operation (for example, tapping) performed on the "Cancel" control 118, the wearable device 100 may display, on the display 12, the golf icon and the texts "golf mode" shown in FIG. 10.

If the user currently wants to input personal exercise data, the user may tap the "Confirm" control 117. If the user currently does not want to input personal exercise data, the user may tap the "Cancel" control 118.

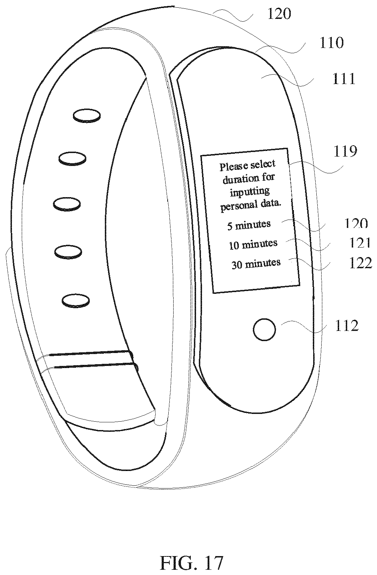

When detecting a user operation performed on the "Confirm" control 117, the wearable device 100 displays, on the display 12, a user interface shown in FIG. 17. The user interface may be used for the user to select a length of a time for inputting personal exercise data. The user interface may include a prompt box 119.

The prompt box 119 may include a "5 minutes" option 120, a "10 minutes" option 121, and a "30 minutes" option 122. The prompt box 119 may further include more or fewer options. This is not limited in this application.

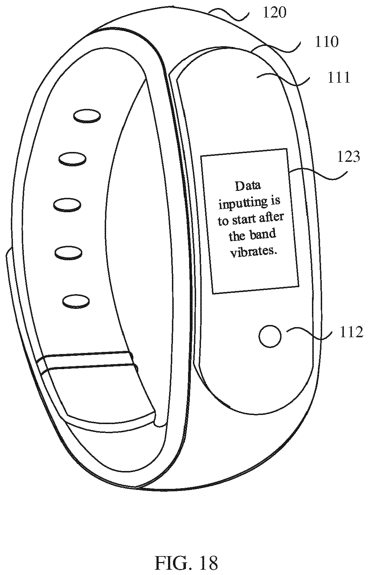

In response to a user operation (for example, tapping) performed on any option in the prompt box 119, the wearable device 100 may display, on the display 12, a user interface shown in FIG. 18. The user interface includes a prompt box 123. The prompt box 123 is configured to notify the user that data inputting is to start after the band vibrates. The prompt box 123 includes prompt content: "Data inputting is to start after the band vibrates.".

When detecting a user operation performed on any option in the prompt box 123, the wearable device 100 displays a user interface and vibrates. After the vibration ends, the wearable device 100 starts to record personal exercise data.

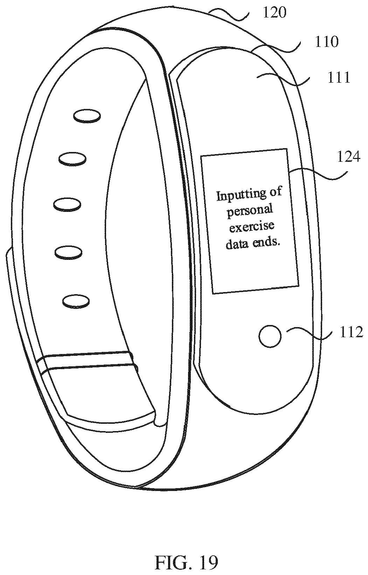

When the wearable device 100 detects that a time length, after inputting of personal exercise data starts, corresponding to an option selected in the prompt box 123 has elapsed, the wearable device 100 may vibrate and display, on the display 12, a user interface shown in FIG. 19. The user interface may be used to notify the user that inputting of personal exercise data ends.

The user interface shown in FIG. 19 includes a prompt box 124. The prompt box 124 is used to notify the user that inputting of personal exercise data ends. The prompt box 124 includes prompt content: "Inputting of personal exercise data ends.".

When the user inputs personal exercise data, that is, when the user performs a golf action, it is not convenient for the user to directly see content on the display 12. The wearable device 100 may notify, through vibration, the user that inputting of personal exercise data ends. In this way, the user can stop performing a golf action after the wearable device 100 vibrates.

A manner of starting, by the wearable device 100, inputting of personal exercise data and a manner of ending inputting of personal exercise data are not limited in this embodiment of this application. In addition to indicating, through vibration, that inputting of personal exercise data starts and inputting of personal exercise data ends, another manner may be alternatively used.

That the electronic device 200 prompts the user to input personal exercise data after the wearable device 100 enables the golf mode for the first time is described below.

In some embodiments, when the electronic device 200 detects that the user enables the golf mode in the "Sports and Health" application, and detects no personal exercise data of the user, the electronic device may prompt the user to input personal exercise data.

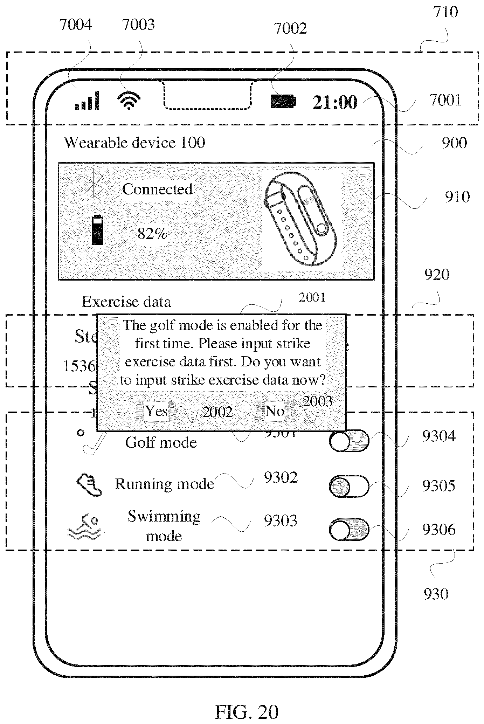

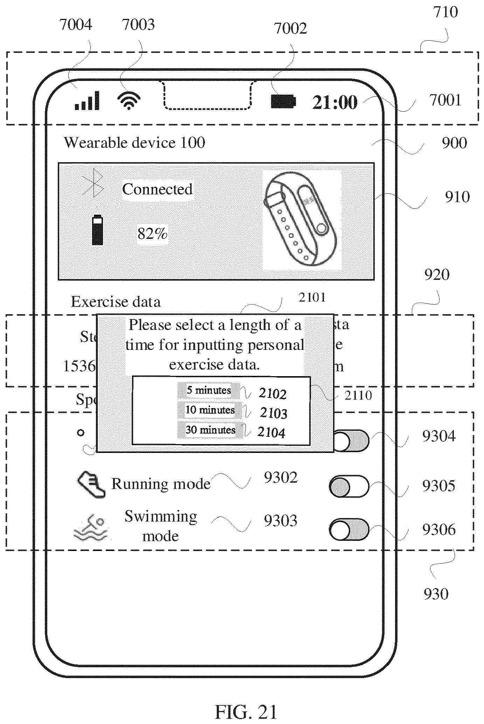

The electronic device 200 may display, on the user interface 900, a prompt box 2001 shown in FIG. 20. The prompt box 2001 includes prompt content. The prompt content is used by the electronic device 200 to prompt the user to input exercise data after the golf mode is enabled. The prompt content may include: "The golf mode is enabled for the first time. Please input strike exercise data first. Do you want to input strike exercise data now?". The prompt box 2001 further includes a control 2002. The control 2002 may be configured to receive a user operation (for example, tapping). In response to the user operation (for example, tapping) performed on the control 2002, the electronic device 200 may display, on the user interface 900, a prompt box 2101 shown in FIG. 21. The prompt box 2001 further includes a control 2003. The control 2003 may be configured to cancel inputting personal exercise data. If the user currently wants to input personal exercise data, the user may tap the control 2002. If the user currently does not want to input personal exercise data, the user may tap the control 2003.

When detecting a user operation performed on the control 2002, the electronic device 200 displays, on the user interface 900, the prompt box 2101 shown in FIG. 21.

As shown in FIG. 21, the prompt box 2101 may include a "5 minutes" option 2102, a "10 minutes" option 2103, and a "30 minutes" option 2104. The prompt box 2101 may further include more or fewer options. This is not limited in this application.

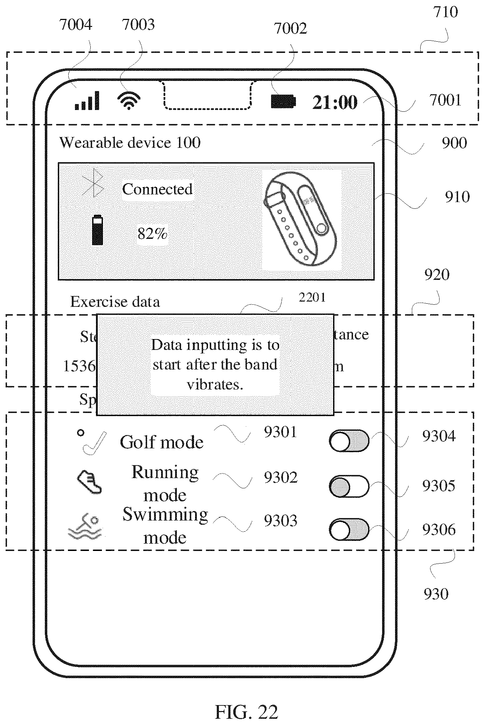

In response to a user operation (for example, tapping) performed on any option in the prompt box 2101, the electronic device 200 may display, on the user interface 900, a prompt box 2201 shown in FIG. 22.

As shown in FIG. 22, the prompt box 2201 is configured to notify the user that data inputting is to start after the band vibrates. The prompt box 2201 includes prompt content: "Data inputting is to start after the band vibrates.".

When detecting a user operation performed on any option in the prompt box 123, the wearable device 100 displays a user interface and vibrates. After the vibration ends, the wearable device 100 starts to record personal exercise data.

That the wearable device 100 calculates a swing action parameter of the user is described below.

After the golf mode is enabled, the acceleration sensor, the gyro sensor, and the sound collector in the wearable device 100 are all in an operating state. The acceleration sensor may be configured to collect acceleration data of the wrist. The gyro sensor may be configured to collect angular velocity data of the wrist. The sound collector may be configured to collect a sound signal.

The wearable device 100 processes the acceleration data and the angular velocity data to obtain an acceleration waveform feature and an angular velocity waveform feature.

The wearable device 100 may obtain, based on the acceleration waveform feature and the angular velocity waveform, a moment corresponding to an up-swing start point, a moment corresponding to an up-swing end point, and a moment corresponding to a down-swing end point.

The wearable device 100 may calculate an up-swing time based on the moment corresponding to the up-swing start point and the moment corresponding to the up-swing end point. The wearable device 100 may calculate a down-swing time based on the moment corresponding to the up-swing end point and the moment corresponding to the down-swing end point.

A swing rhythm of the user may be calculated based on a ratio of the up-swing time to the down-swing time.

Within the up-swing time, the wearable device 100 may calculate, based on an integral of an acceleration change in a vertical direction, a moving distance S of the wrist of the user in the vertical direction in the up-swing stage.

Within the up-swing time, the wearable device 100 may calculate a rotation angle Q of the wrist of the user in the up-swing stage based on an integral of an angular velocity change within the up-swing time.

The wearable device 100 may calculate a swing speed based on an acceleration of the wrist at each moment.

Specifically, for detailed descriptions of calculating the swing speed, refer to the descriptions of the swing speed in the explanations of the terms. Details are not described herein again in this application.

The wearable device 100 may determine, based on a trained Gaussian mixture model, whether a sound signal is a sound signal generated by a strike or a sound signal generated by a whiff, and further count a quantity of strikes of the user.

A principle of the Gaussian mixture model and a training process of the Gaussian model are described below.

Gaussian mixture model: The Gaussian mixture model is a probability statistics model, and may depict a statistical distribution of feature parameters of sound signals by using a weighted linear combination of a Gaussian probability density function. A mean vector of each Gaussian distribution in the Gaussian mixture model may represent a category of sound signals generated by strikes or whiffs. A covariance matrix of each Gaussian distribution may represent variability of this category of sound wave signals. A sound signal generated by a strike and a sound signal generated by a whiff are different in feature parameter values (such as an amount of energy, frequencies, and peak values). Therefore, distributions of different categories of sound signals may be established based on these different feature parameter values.

These different distributions may be used to distinguish a sound signal generated by a strike and a sound signal generated by a whiff.

In this embodiment of this application, each individual Gaussian distribution in the trained Gaussian mixture model used by the wearable device 100 represents a distribution of sound signals generated by strikes. Based on differences of feature parameter values, sound signals may be classified into a sound signal generated by a strike and a sound signal generated by a whiff. Further, sound signals generated by strikes may be further classified into a plurality of categories based on differences of feature parameter values. A plurality of Gaussian distributions included in the Gaussian mixture model are distributions of different categories of sound signals generated by strikes. The wearable device 100 may perform similarity matching calculation on a feature parameter of a collected sound signal and the trained Gaussian mixture model, to determine whether the sound signal is a sound signal generated by a strike.

In a possible implementation, the wearable device 100 may perform audio signal processing on a sound signal generated by a strike, to frame the sound signal and extract a multi-dimensional feature parameter. The multi-dimensional feature parameter may have M dimensions, and may include feature parameters such as an amount of energy, a frequency, and a peak value of the sound signal. M is a positive integer. For a process of the audio signal processing, refer to an audio signal processing method in the conventional technology. Details are not described in this embodiment of this application. A parameter included in the multi-dimensional feature parameter is not specifically limited in this embodiment of this application.

The Gaussian mixture model may include N separate Gaussian distributions, where one Gaussian distribution may represent a distribution of one type of sound signal generated by a strike. The Gaussian mixture model may be represented through linear weighting on N M-dimensional Gaussian probability density functions, and an expression may be shown in a formula (1):

$$(X) = \sum_{i=1}^{N} wi \times Gi(X) \qquad\qquad \text{Formula (1)},$$

where

$X = (x1, ..., xj, ..., xM)$ and may represent an M-dimensional feature parameter of the sound signal, $\omega i$ may represent a weight of an $i^{th}$ Gaussian probability density function, $\sum_{i=1}^{N} wi = 1$, Gi(X)(i = 1, ..., N) may represent a Gaussian probability density function of an $i^{th}$ Gaussian distribution, and an expression of Gi(X) may be shown in a formula (2):

$$Gi(X) = \frac{1}{(2\pi)^{\frac{M}{2}} |C_i|^{\frac{1}{2}}} \exp\{-\frac{1}{2}(X - Ui)^T C_i^{-1}(X - Ui)\} \qquad\qquad \text{Formula (2)},$$

where

U$i$ and C$i$ are a mean vector and a covariance matrix of the $i^{th}$ Gaussian probability density function respectively. When obtaining the M-dimensional feature parameter of the sound signal generated by the strike, the wearable device 100 may train the Gaussian mixture model by using the M-dimensional feature parameter, to obtain an optimal solution of a Gaussian mixture model parameter $\lambda = \{\omega i, \mu i, Ci\}$.

In a possible implementation, the wearable device 100 may estimate the optimal solution of the parameter $\lambda$ through iterative calculation of an expectation maximization algorithm.

A specific Gaussian distribution, in the Gaussian mixture model, to which a distribution of a sound signal used for training the Gaussian mixture model belongs is unknown. The expectation maximization algorithm first assumes an initial value of the parameter $\lambda$, that is, first assumes that information about the specific Gaussian distribution, in the Gaussian mixture model, to which the distribution of the sound signal used for training belongs is known. Then an expectation step is performed. The expectation step indicates that a specific Gaussian distribution, in the Gaussian mixture model, that generates the sound signal used for training is determined through calculation based on a value of the parameter $\lambda$, that is, data used for training is classified. Then a maximization step is performed based on a classification result. The maximization step indicates that maximum likelihood estimation is performed on the parameter $\lambda$, that is, the value of the parameter $\lambda$ is updated. The expectation step and the maximization step are repeated. When the value of the parameter $\lambda$ converges, the wearable device 100 may stop the training process, and use the last value obtained by updating the parameter $\lambda$ as an optimal solution of the parameter $\lambda$. In this way, a trained Gaussian mixture model can be obtained.

That the value of the parameter $\lambda$ converges may indicate that a difference between parameters $\lambda$ updated in two consecutive maximization steps is less than a specified threshold, that is, it may be considered that the value of the parameter $\lambda$ is stable and unchanged.

That the wearable device 100 determines, based on the Gaussian mixture model, whether the user is performing a strike or a whiff is described below.

When obtaining the trained Gaussian mixture model, the wearable device 100 may distinguish, by using the trained Gaussian mixture model, a sound signal generated by a strike from a sound signal generated by a whiff in sound signals collected by the microphone. For example, the wearable device 100 performs audio signal processing on a sound signal collected by the microphone, to obtain an M-dimensional feature parameter $X = (x1, ..., xj, ..., xM)$ of a specific frame of sound signal. The wearable device 100 may perform similarity matching calculation on this frame of sound signal and the trained Gaussian mixture model based on the feature parameter. When the M-dimensional feature parameter of this frame of sound signal is known, the wearable device 100 may calculate a posterior probability that this frame of sound signal is generated by an $i^{th}$ Gaussian distribution in the Gaussian mixture model. A specific calculation formula may be shown in formula (3):

$$p(i|X) = \frac{wiGi(X)}{P(X)} \qquad \text{Formula (3)}$$

In this case, a sum of posterior probabilities that this frame of sound signal is generated by N Gaussian distributions in the Gaussian mixture model is as follows: $Q = \sum_{i=1}^{N} P(i|X)$ . The sum $Q$ of the probabilities may represent a similarity between this frame of sound signal and the Gaussian mixture model. The wearable device 100 may set a matching threshold. When the sum $Q$ of the posterior probabilities is greater than the matching threshold, the wearable device 100 may determine that this frame of sound signal is a sound signal generated by a strike. Otherwise, the wearable device 100 may determine that this frame of sound signal is a sound signal generated by a whiff. A value of the matching threshold is not specifically limited in this embodiment of this application. The foregoing similarity matching calculation method is not limited in this embodiment of this application, and may alternatively be another similarity matching method.

Further, the wearable device 100 may calculate, based on a sound signal generated by a strike, a total quantity of strikes of the user in one process of playing golf, that is, a total quantity of frames of sound signals generated by strikes.

When performing framing on a sound signal collected when the user plays golf, the wearable device 100 may determine a length of one frame of sound signal based on a common strike frequency of a user. For example, the length of one frame of sound signal may be 300 milliseconds. When determining that a frame of sound signal is a sound signal generated by a strike, the wearable device 100 may determine that the user performs one strike, that is, a quantity of frames of sound signals generated by strikes is a total quantity of strikes of the user in one process of playing golf.

The Gaussian mixture model is a weighted linear combination of a plurality of Gaussian probability density functions, and a linear weighted combination of several Gaussian probability density functions may be used to approximate any distribution. Therefore, the Gaussian mixture model may be used to describe a distribution of sound signals generated by strikes. In this way, the wearable device 100 can determine, by using the Gaussian mixture model, whether the user performs a strike. With reference to an action, of the wrist of the user, that is obtained by analyzing the acceleration data and the angular velocity data, the wearable device 100 may detect an overall action (for example, a full-swing strike, a half-swing strike, a whiff, or a ground hit) of the user when the user plays golf, to better assist the user in improving an ability to play golf.

A method for processing a sound signal to determine whether the user performs a strike is not limited in this embodiment of this application. In addition to the algorithm of the Gaussian mixture model, another sound signal identification algorithm, such as an algorithm of a hidden Markov model or a neural network algorithm, may be used.

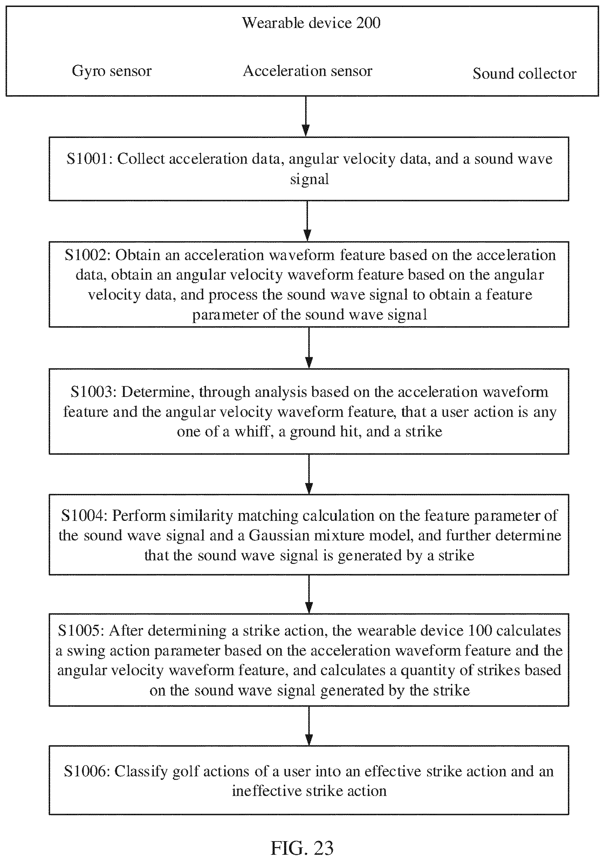

FIG. 23 is a flowchart of a detection method for detecting an action of a user when the user plays golf.

As shown in FIG. 23, the method may include the following steps.

S 1001: A wearable device 100 collects acceleration data, angular velocity data, and a sound signal.

The wearable device 100 is worn on the wrist of a user. After a golf mode is enabled, an acceleration sensor in the wearable device 100 collects acceleration data of the wrist, a gyro sensor collects angular velocity data of the wrist, and a sound collector collects a sound signal during golf sports.

S1002: The wearable device 100 obtains an acceleration waveform feature based on the acceleration data, obtains an angular velocity waveform feature map based on the angular velocity data, and processes the sound signal to obtain a feature parameter of the sound signal.

That the wearable device 100 processes the sound signal includes framing the sound signal and extracting a feature parameter. The wearable device 100 may determine a length of one frame of sound signal based on a common strike frequency of a user. For example, usually, it takes 300 milliseconds for a user to perform a strike. In this case, the wearable device 100 may preset 300 milliseconds to segment the sound signal. In this way, if determining that one frame of sound signal is a sound signal generated by a strike, the wearable device 100 may determine that the user performs one strike.

The wearable device 100 may extract a feature parameter of each frame of sound signal. The feature parameter may include an M-dimensional feature parameter, such as an amount of energy, a frequency, and a peak value, of the frame of sound signal. M is a positive integer greater than or equal to 1.

S1003: The wearable device 100 determines, through analysis based on the acceleration waveform feature and the angular velocity waveform feature, that a user action is any one of a whiff, a ground hit, and a strike.

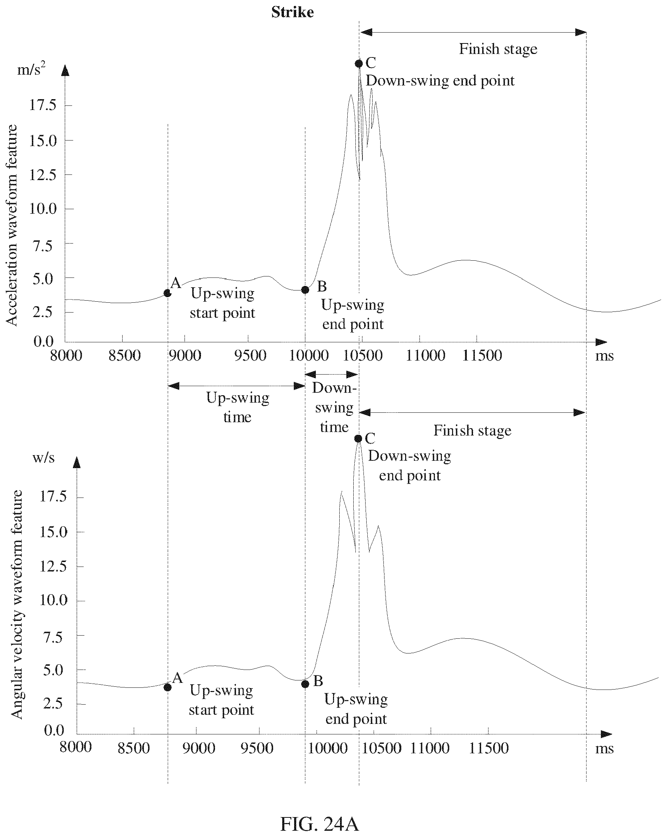

FIG. 24A is an example schematic diagram of an acceleration waveform feature and an angular velocity waveform feature in the case of a strike.

The wearable device 100 may obtain features, such as a quantity of peaks/troughs, a peak value, and a peak-trough difference, from an acceleration waveform feature map and an angular velocity waveform feature map. Because a club head of a golf club collides with a ball, back-and-forth fluctuations occur between a plurality of peak values. It may be determined that a strike action occurs when peaks and troughs appear in the acceleration waveform feature map and the angular velocity waveform feature map, a quantity of peaks or troughs is greater than a first quantity threshold (for example, a quantity is 2), a maximum peak value is greater than a first peak threshold, a difference between the maximum peak value and an adjacent minimum trough value is greater than a first difference threshold, and an acceleration and an angular velocity gradually decrease. The wearable device 100 may determine a down-swing end point based on a moment (a first moment) corresponding to the maximum peak value. In this case, the down-swing end point is a strike point, and it is determined that a time at which the strike point appears is the first moment.

The wearable device 100 may determine, in any one of the following manners, that the user action is a strike.

Manner 1: The wearable device 100 determines that the user action is a strike action when the wearable device 100 learns, from the acceleration waveform feature map and the angular velocity waveform feature map, that the quantity of peaks or troughs is greater than the first quantity threshold (for example, the quantity is 2), the maximum peak value is greater than the first peak threshold, the difference between the maximum peak value and the adjacent minimum trough value is greater than the first difference threshold, and a time in which the acceleration decreases to a first acceleration threshold is greater than a first time threshold and/or a time in which the angular velocity decreases to a first angular velocity threshold is greater than a second time threshold.

Manner 2: The wearable device 100 determines that the user action is a strike action when the wearable device 100 learns, from the acceleration waveform feature map and the angular velocity waveform feature map, that the quantity of peaks or troughs is greater than the first quantity threshold (for example, the quantity is 2), the maximum peak value is greater than the first peak threshold, and a time in which the acceleration decreases to a first acceleration threshold is greater than a first time threshold and/or a time in which the angular velocity decreases to a first angular velocity threshold is greater than a second time threshold.

Manner 3: The wearable device 100 determines that the user action is a strike action when the wearable device 100 learns, from the acceleration waveform feature map and the angular velocity waveform feature map, that the quantity of peaks or troughs is greater than the first quantity threshold (for example, the quantity is 2), the maximum peak value is greater than the first peak threshold, and a time in which the acceleration decreases to a first acceleration threshold is greater than a first time threshold and/or a time in which the angular velocity decreases to a first angular velocity threshold is greater than a second time threshold.

Manner 4: The wearable device 100 determines that the user action is a strike action when the wearable device 100 learns, from the acceleration waveform feature map and the angular velocity waveform feature map, that the quantity of peaks or troughs is greater than the first quantity threshold (for example, the quantity is 2), and a time in which the acceleration decreases to a first acceleration threshold is greater than a first time threshold and/or a time in which the angular velocity decreases to a first angular velocity threshold is greater than a second time threshold.

Alternatively, the wearable device 100 may determine, in another manner, that the user action is a strike action. This is not limited in this application.

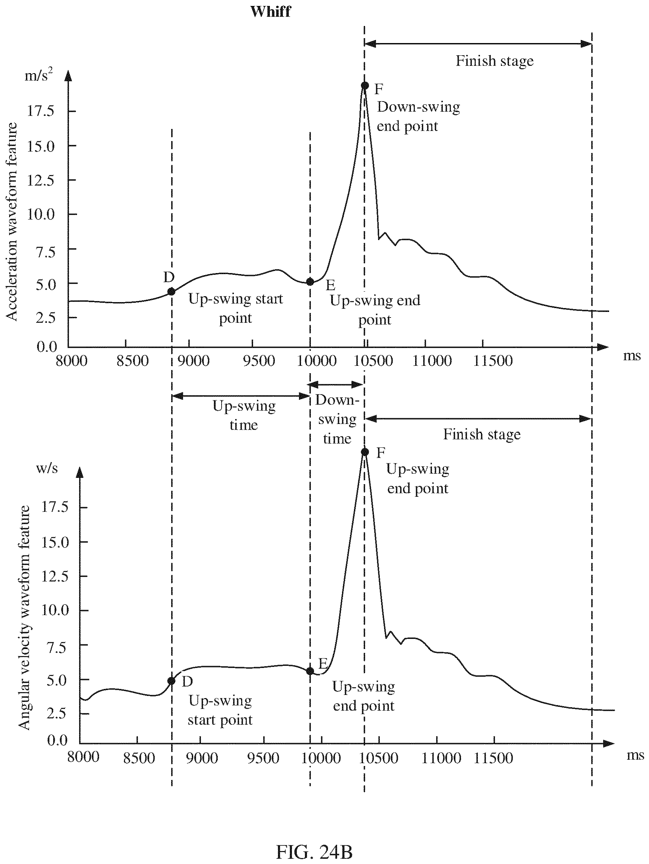

FIG. 24B is an example schematic diagram of an acceleration waveform feature and an angular velocity waveform feature in the case of a whiff.

The wearable device 100 may obtain features, such as a quantity of peaks/troughs, a peak value, and a peak-trough difference, from an acceleration waveform feature map and an angular velocity waveform feature map. It may be determined that a whiff action occurs when peaks appear in the acceleration waveform feature map and the angular velocity waveform feature map, a quantity of peaks is less than a first quantity threshold (for example, a quantity is 2), a maximum peak value is less than a first peak threshold, a difference between the maximum peak value and an adjacent minimum trough value is less than a first difference threshold, and an acceleration and an angular velocity gradually decrease. The wearable device 100 may determine a down-swing end point based on a moment (a first moment) corresponding to the maximum peak value, and determine that a time at which the strike point appears is the first moment.

The wearable device 100 may determine, in any one of the following manners, that the user action is a whiff.

Manner 1: When the wearable device 100 learns, from the acceleration waveform feature map and the angular velocity waveform feature map, that the quantity of peaks or troughs is less than the first quantity threshold (for example, the quantity is 2), the maximum peak value is less than the first peak threshold, the difference between the maximum peak value and the adjacent minimum trough value is less than the first difference threshold, and a time in which the acceleration decreases to a first acceleration threshold is greater than a first time threshold and/or a time in which the angular velocity decreases to a first angular velocity threshold is greater than a second time threshold, the wearable device 100 determines that the user action is a whiff action.

Manner 2: When the wearable device 100 learns, from the acceleration waveform feature map and the angular velocity waveform feature map, that the quantity of peaks or troughs is less than the first quantity threshold (for example, the quantity is 2), the difference between the maximum peak value and the adjacent minimum trough value is less than the first difference threshold, and a time in which the acceleration decreases to a first acceleration threshold is greater than a first time threshold and/or a time in which the angular velocity decreases to a first angular velocity threshold is greater than a second time threshold, the wearable device 100 determines that the user action is a whiff action.

Manner 3: When the wearable device 100 learns, from the acceleration waveform feature map and the angular velocity waveform feature map, that the quantity of peaks or troughs is less than the first quantity threshold (for example, the quantity is 2), the maximum peak value is less than the first peak threshold, and a time in which the acceleration decreases to a first acceleration threshold is greater than a first time threshold and/or a time in which the angular velocity decreases to a first angular velocity threshold is greater than a second time threshold, the wearable device 100 determines that the user action is a whiff action.

Manner 4: When the wearable device 100 learns, from the acceleration waveform feature map and the angular velocity waveform feature map, that the quantity of peaks or troughs is less than the first quantity threshold (for example, the quantity is 2), and a time in which the acceleration decreases to a first acceleration threshold is greater than a first time threshold and/or a time in which the angular velocity decreases to a first angular velocity threshold is greater than a second time threshold, the wearable device 100 determines that the user action is a whiff action.

Alternatively, the wearable device 100 may determine, in another manner, that the user action is a whiff action. This is not limited in this application.

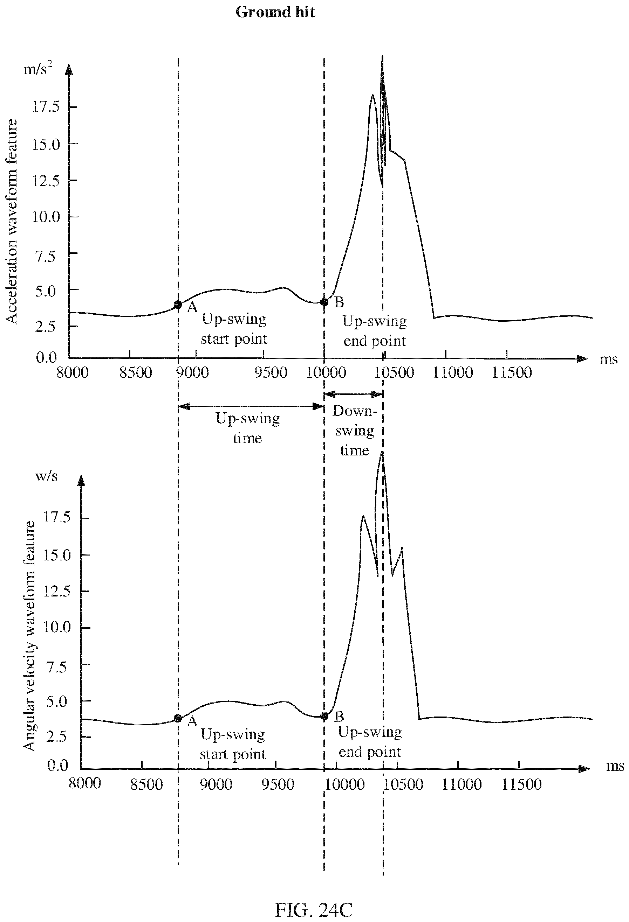

FIG. 24C is an example schematic diagram of an acceleration waveform feature and an angular velocity waveform feature in the case of a ground hit.

Herein, the ground hit means that the user operates the golf club, and the golf club hits the grass without colliding with a ball due to misjudgment.

The wearable device 100 may obtain features, such as a quantity of peaks/troughs, a peak value, and a peak-trough difference, from an acceleration waveform feature map and an angular velocity waveform feature map. It may be determined that a ground hit action occurs when peaks and troughs appear in the acceleration waveform feature map and the angular velocity waveform feature map, a quantity of peaks or troughs is greater than a first quantity threshold (for example, a quantity is 2), a maximum peak value is greater than a first peak threshold, and a difference between the maximum peak value and an adjacent minimum trough value is greater than a first difference threshold, but an acceleration and an angular velocity sharply decrease to a minimum value. The wearable device 100 may determine a down-swing end point based on a moment (a first moment) corresponding to the maximum peak value.

The wearable device 100 may determine, in any one of the following manners, that the user action is a ground hit.

Manner 1: The wearable device 100 determines that the user action is a ground hit action when the wearable device 100 learns, from the acceleration waveform feature map and the angular velocity waveform feature map, that the quantity of peaks is greater than the first quantity threshold (for example, the quantity is 2), the maximum peak value is greater than the first peak threshold, the difference between the maximum peak value and the adjacent minimum trough value is greater than the first difference threshold, and a time in which the acceleration decreases to a first acceleration threshold is less than a first time threshold and/or a time in which the angular velocity decreases to a first angular velocity threshold is greater than a second time threshold.

Manner 2: The wearable device 100 determines that the user action is a ground hit action when the wearable device 100 learns, from the acceleration waveform feature map and the angular velocity waveform feature map, that the quantity of peaks is greater than the first quantity threshold (for example, the quantity is 2), the difference between the maximum peak value and the adjacent minimum trough value is greater than the first difference threshold, and a time in which the acceleration decreases to a first acceleration threshold is less than a first time threshold and/or a time in which the angular velocity decreases to a first angular velocity threshold is greater than a second time threshold.

Manner 3: The wearable device 100 determines that the user action is a ground hit action when the wearable device 100 learns, from the acceleration waveform feature map and the angular velocity waveform feature map, that the quantity of peaks is greater than the first quantity threshold (for example, the quantity is 2), the maximum peak value is greater than the first peak threshold, and a time in which the acceleration decreases to a first acceleration threshold is less than a first time threshold and/or a time in which the angular velocity decreases to a first angular velocity threshold is greater than a second time threshold.

Manner 4: The wearable device 100 determines that the user action is a ground hit action when the wearable device 100 learns, from the acceleration waveform feature map and the angular velocity waveform feature map, that the quantity of peaks is greater than the first quantity threshold (for example, the quantity is 2), and a time in which the acceleration decreases to a first acceleration threshold is less than a first time threshold and/or a time in which the angular velocity decreases to a first angular velocity threshold is greater than a second time threshold.

Alternatively, the wearable device 100 may determine, in another manner, that the user action is a ground hit action. This is not limited in this application.

S1004: The wearable device 100 performs similarity matching calculation on the feature parameter of the sound signal and a Gaussian mixture model, and further determine that the sound signal is generated by a strike.

In step S1003, the wearable device 100 has determined, through analysis based on the acceleration waveform feature and the angular velocity waveform feature, that the user action is any one of a whiff, a ground hit, and a strike. However, at a down-swing end point, the user operates a club head of the golf club to prepare for striking a ball, but the club head does not hit the ball due to a mistake. However, in this case, a speed of the club head is quite high. If the wrist of the user shakes or the wearable device 100 worn by the user is loose, the wearable device 100 may also determine, through analysis based on the quantity of peaks and a peak value in the acceleration waveform feature and the angular velocity waveform feature, that the user is performing a strike action. Based on the foregoing impact, the wearable device 100 may mistake a whiff action of the user as a strike action.

To eliminate an error, the wearable device 100 collects a sound signal, performs similarity matching calculation on a feature parameter of a sound signal at a first moment and a Gaussian mixture model, and determines that the sound signal is generated by a strike. This eliminates misjudgment.

When the user operates the club head to hit the ball, the club head collides with the ball, and a loud sound is generated. A sound signal collected by the wearable device 100 when the user performs a strike action is greatly different from a sound signal collected by the wearable device when the user performs a whiff action. In a spectrum graph of sound signals collected by the wearable device 100 at the first moment, a peak value and an amount of energy of a sound signal generated by a strike are far greater than a peak value and an amount of energy of a sound signal generated by a whiff. Based on this difference, the wearable device 100 can distinguish whether the user is performing a strike action or a whiff action.

Specifically, the Gaussian mixture model may include N separate Gaussian distributions. Each Gaussian distribution may represent a category of one type of sound signal generated by a strike. When an M-dimensional feature parameter of one frame of sound signal is known, the wearable device 100 may calculate, based on the formula (3), a posterior probability that this frame of sound signal is generated by an $1^{th}$ Gaussian distribution in the Gaussian mixture model. In this way, the wearable device 100 can obtain a sum $Q$ of posterior probabilities that this frame of sound signal is generated by N Gaussian distributions in the Gaussian mixture model. $Q = \sum_{i=1}^{N} P(i|X)$, and may represent a similarity between this frame of sound signal and a strike Gaussian mixture model.

The wearable device 100 may set a matching threshold. When the sum Q of the posterior probabilities is greater than the matching threshold, the wearable device 100 may determine that this frame of sound signal is a sound signal generated by a strike. A value of the matching threshold is not specifically limited in this embodiment of this application.

In some embodiments, alternatively, the wearable device 100 may directly perform similarity matching calculation based on the feature parameter of the sound signal and the Gaussian mixture model, and determine that the sound signal is generated by a strike.

S 1005: After determining a strike action, the wearable device 100 calculates a swing action parameter based on the acceleration waveform feature and the angular velocity waveform feature, and the wearable device 100 calculates a quantity of strikes based on the sound signal generated by the strike.

The swing action parameter may be, for example, an up-swing time, a down-swing time, a swing speed, or a swing rhythm. The swing action parameter may further include more other parameters. This is not limited in this application.

The wearable device 100 may obtain, based on the acceleration waveform feature and the angular velocity waveform, a moment corresponding to an up-swing start point, a moment corresponding to an up-swing end point, and a moment corresponding to a down-swing end point.

The wearable device 100 may calculate an up-swing time based on the moment corresponding to the up-swing start point and the moment corresponding to the up-swing end point. The wearable device 100 may calculate a down-swing time based on the moment corresponding to the up-swing end point and the moment corresponding to the down-swing end point.

A swing rhythm of the user may be calculated based on a ratio of the up-swing time to the down-swing time.

The wearable device 100 may calculate a swing speed based on an acceleration of the wrist at each moment.

Specifically, for detailed descriptions of calculating the swing speed, refer to the descriptions of the swing speed in the explanations of the terms. Details are not described herein again in this application.

The wearable device 100 may obtain a total quantity of ground hits of the user based on the acceleration waveform feature and the angular velocity waveform feature.

If the wearable device 100 determines that the sound signal is generated by a strike, the wearable device 100 may increment a quantity of strikes of the user by 1 in a process of playing golf by the user.

If the wearable device 100 determines that the sound signal is a sound signal generated by a whiff, the wearable device 100 may also calculate a quantity of whiffs of the user, and increment a quantity of whiffs of the user by 1 in a process of playing golf by the user.

By analogy, the wearable device 100 may calculate a total quantity of strikes, a total quantity of whiffs, and a total quantity of ground hits of the user in one golf exercise.

In some embodiments, the wearable device 100 may consider a whiff action of the user and a ground hit action of the user as ineffective actions, and consider a strike action of the user as an effective action. The wearable device 100 may calculate, based on a total quantity of whiffs and a total quantity of ground hits of the user in one golf exercise, a quantity of ineffective actions of the user in one golf exercise. The wearable device 100 may calculate, based on a total quantity of strikes of the user in one golf exercise, a quantity of effective actions of the user in one golf exercise.

S1006: The wearable device 100 classifies golf actions of the user into an effective strike action and an ineffective strike action.

The wearable device 100 may classify golf actions of the user into an effective strike action and an ineffective strike action based on an analysis result, of whether a strike action is performed, that is determined with reference to an action of the wrist of the user and a sound signal collected at a same moment.

Effective strike actions may include a full-swing strike and a half-swing strike.

Half-swing strike: The half-swing strike includes two aspects: a half swing and a strike. For a specific explanation of the half swing, refer to the explanations of the terms. Details are not described herein again in this application.

The wearable device 100 may determine a half-swing strike action in any one of the following three manners.

Manner 1 Within an up-swing time, the wearable device 100 determines that a rotation angle Q of the wrist is less than or equal to a first preset angle. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a half-swing strike action.

Manner 2: Within an up-swing time, the wearable device 100 determines that a moving distance S of the wrist in a vertical direction is less than or equal to a first preset distance. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a half-swing strike action.

Manner 3: Within an up-swing time, the wearable device 100 determines that a rotation angle Q of the wrist is less than or equal to a first preset angle and a moving distance S of the wrist in a vertical direction is less than or equal to a first preset distance. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a half-swing strike action.

A method for determining, by the wearable device 100, that the user is performing a half-swing strike action may alternatively be another manner. This is not limited herein in this application.

Full-swing strike: The full-swing strike includes two aspects: a full swing and a strike. For a specific explanation of the full swing, refer to the explanations of the terms. Details are not described herein again in this application.

The wearable device 100 may determine a full-swing strike action in any one of the following three manners.

Manner 1 Within an up-swing time, the wearable device 100 determines that a rotation angle Q of the wrist is greater than a first preset angle. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a full-swing strike action.

Manner 2: Within an up-swing time, the wearable device 100 determines that a moving distance S of the wrist in a vertical direction is greater than a first preset distance. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a full-swing strike action.

Manner 3: Within an up-swing time, the wearable device 100 determines that a rotation angle Q of the wrist is greater than a first preset angle and a moving distance S of the wrist in a vertical direction is greater than a first preset distance. In addition, the wearable device 100 obtains a feature parameter of a frame of sound signal at a moment of a down-swing end point. The wearable device 100 performs similarity matching calculation on the feature parameter of the frame of sound signal and the Gaussian mixture model, to obtain a first similarity. The Gaussian mixture model has a preset first threshold. If the first similarity is greater than the first threshold, it is determined that the sound signal is generated by a strike. In this case, the wearable device 100 determines that the user action is a full-swing strike action.

A method for determining, by the wearable device 100, that the user is performing a full-swing strike action may alternatively be another manner. This is not limited herein in this application.

Effective strike actions are not limited to the half-swing strike and the full-swing strike, and may further include more other categories. This is not limited herein in this application.

Ineffective actions may include a ground hit and a whiff.

Ground hit: The wearable device 100 may obtain features, such as a quantity of peaks and a peak value, from an acceleration waveform feature map and an angular velocity waveform feature map. It may be determined that a ground hit action occurs when peaks appear in the acceleration waveform feature map and the angular velocity waveform feature map, and there is more than one peak, but an acceleration and an angular velocity sharply decrease to a minimum value.

For details, refer to the descriptions in S 1003. Details are not described herein again in this application.

Miss: The wearable device 100 collects a sound signal, and processes a frame of sound signal at a down-swing end point, to obtain a feature parameter of the frame of sound signal. The wearable device 100 performs similarity matching calculation on the feature parameter of the frame of sound signal and the Gaussian mixture model, to obtain a first similarity. The Gaussian mixture model has a preset first threshold. If the first similarity is less than the first threshold, it is determined that the sound signal is generated by a whiff.

The wearable device 100 determines, through analysis, that the user is performing a strike action, and calculates a swing action parameter of the user based on acceleration data and angular velocity data. The method implements detection on a strike action of the user, and helps improve stability of a strike rhythm of the user.

Specifically, the wearable device 100 may detect a strike action of the user within a data input time (for example, 5 minutes), collects statistics on a swing action parameter of the user based on exercise data, and provides a suggestion on a swing action of the user with reference to each swing action parameter of the user. This helps improve stability of a swing action of the user.

For example, the wearable device 100 detects, within a data entry time (for example, 5 minutes), that the user performs 12 golf swings in total, including eight effective strike actions (full-swing strikes and half-swing strikes) and four ineffective strike actions (whiffs and ground hits). The wearable device 100 determines, through calculation, that strike stability of the user is 67%. The strike stability of the user is low, and the wearable device 100 may advise the user to improve the strike stability.

The wearable device 100 may also segment each golf swing of the user, to obtain swing action parameters such as an up-swing time, a down-swing time, and a swing rhythm of the user.

For example, the wearable device 100 detects, within a data entry time (for example, 5 minutes), that the user performs 12 golf swings in total, including eight effective strike actions (full-swing strikes and half-swing strikes): four full-swing strikes and four half-swing strikes. The wearable device 100 may collect statistics on a swing action parameter (an up-swing time, a down-swing time, a swing rhythm, and the like) of each half-swing strike. A standard value of an exercise parameter is preset on the wearable device 100. The wearable device 100 may collect statistics on a difference between an up-swing time and a standard value of an up-swing time in each half-swing strike, a difference between a down-swing time and a standard value of an up-swing time in each half-swing strike, and a difference between a swing rhythm and a standard value of a swing rhythm in each of four half-swing strikes, so as to determine half-swing strike stability of the user. The wearable device 100 may also collect statistics on a swing action parameter (an up-swing time, a down-swing time, a swing rhythm the like) of each full-swing strike. A standard value of an exercise parameter is preset on the wearable device 100. The wearable device 100 may collect statistics on a difference between an up-swing time and a standard value of an up-swing time in each full-swing strike, a difference between a down-swing time and a standard value of an up-swing time in each full-swing strike, and a difference between a swing rhythm and a standard value of a swing rhythm in each of four full-swing strike, so as to determine full-swing strike stability of the user.

In this way, the wearable device 100 collects statistics on a swing action parameter in each effective strike action of the user, to determine swing stability of the user. In addition, the wearable device 100 may detect standardization of a swing action of the user, to help the user correct a golf action, and help improve stability of a strike rhythm of the user.

The wearable device 100 may display the swing action parameter of the user, and the swing action parameter of the user may also be displayed on the electronic device 200.

That the wearable device 100 displays a swing action parameter of golf sports is described below.

In some embodiments, when obtaining exercise data of playing golf by the user, the wearable device 100 may display, on the display 12, exercise data that is obtained last time when the golf mode is enabled.

That the wearable device 100 displays a swing action parameter in a half-swing strike action is used below as an example for description.

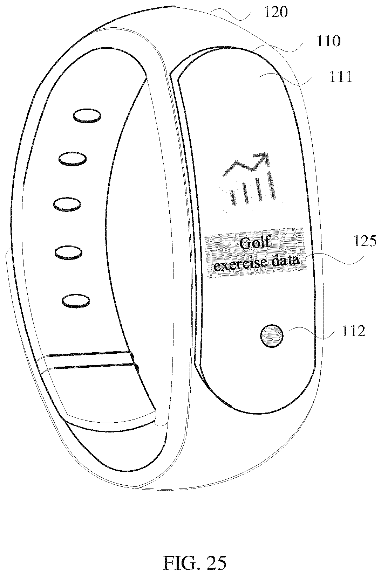

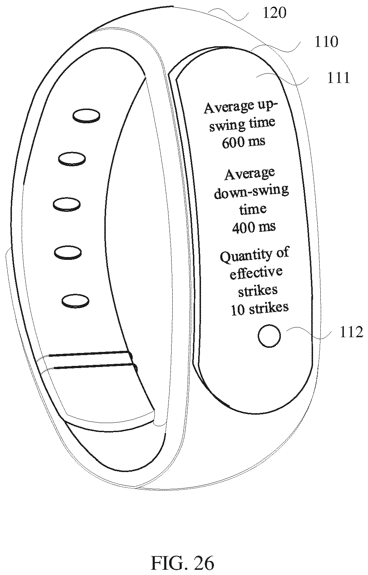

The wearable device 100 may include a plurality of applications, for example, an application "golf exercise data" shown in FIG. 25. In response to a user operation (for example, tapping) performed on a "golf exercise data" icon 125, the wearable device 100 may display an application interface shown in FIG. 26.

A swing action parameter displayed on the application interface shown in FIG. 26 is calculated by the wearable device 100 based on exercise data of the user that is obtained within personal exercise data input duration (for example, 5 minutes). The application interface may include: an average up-swing time: 600 ms; an average down-swing time: 400 ms; and a quantity of effective strikes: 10 strikes.

Content displayed on the application interface of the wearable device 100 may further include more or less exercise data. This is not limited in this embodiment of this application.

Because an area of the display 12 of the wearable device 100 is limited, swing action parameters displayed on the wearable device 100 are also limited. Therefore, more swing action parameters and suggestions may be displayed on the electronic device 200 that establishes a connection to the wearable device 100.

That the electronic device 200 displays a swing action parameter of golf sports is described below.

That the electronic device 200 displays a swing action parameter in a half-swing strike action is used below as an example for description.

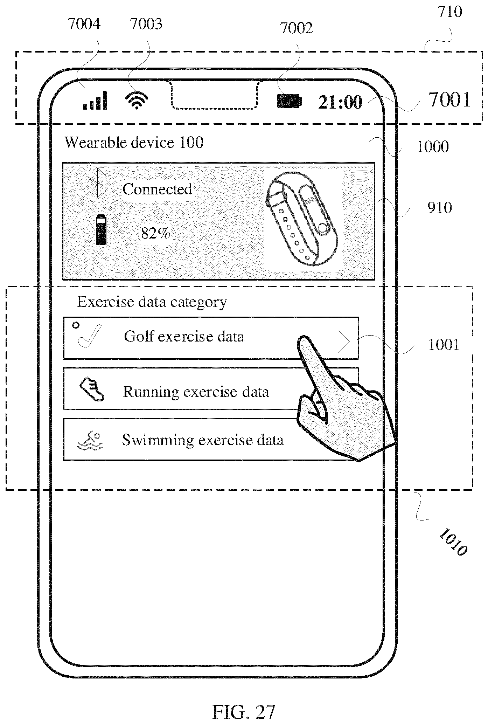

The electronic device 200 may display an application interface 1000 shown in FIG. 27. The application interface 1000 may include a status bar 710, a device status bar 910, and an exercise data bar 1010 corresponding to a sports mode.

The exercise data bar 1010 corresponding to the sports mode may include a golf exercise data option 1001, a running exercise data option, and a swimming exercise data option.

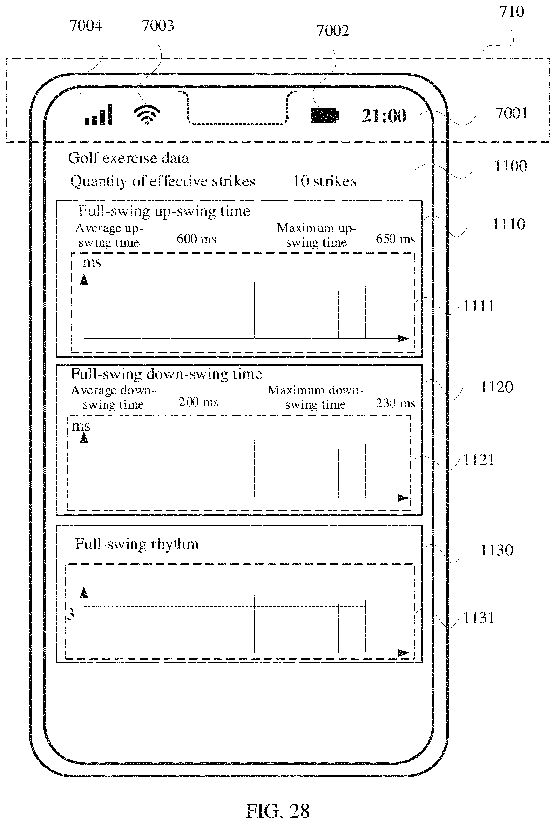

In response to a user operation (for example, tapping) performed on the golf exercise data option 1001, the electronic device 200 may display an application interface 1100 shown in FIG. 28. The application interface 1100 may include a status bar 710, an up-swing time parameter bar 1110, a down-swing time parameter bar 1120, and a swing rhythm parameter bar 1130.

The up-swing time parameter bar 1110 may include: an average up-swing time, where the average up-swing time is 600 ms; a maximum up-swing time, where the maximum up-swing time is 650 ms; and an up-swing time chart 1111.

The up-swing time chart 1111 records an up-swing time corresponding to an up-swing stage in each half-swing strike action of the user. A time of each up-swing stage of the user can be clearly learned from the up-swing time chart 1111.

The down-swing time parameter bar 1120 includes: an average down-swing time, where the average down-swing time is 200 ms; a maximum down-swing time, where the maximum down-swing time is 230 ms; and a down-swing time chart 1121.

The down-swing time chart 1121 records a down-swing time corresponding to a down-swing stage in each half-swing strike action of the user. A time of each down-swing stage of the user can be clearly learned from the down-swing time chart 1121.

The swing rhythm parameter bar 1130 includes a swing rhythm chart 1131.

The swing rhythm chart 1131 records a ratio of an up-swing time and a down-swing time of the user in each half-swing strike action of the user.

A swing rhythm of a good golfer is approximately 3:1. A swing rhythm of the user can be clearly learned from the down-swing time chart 1121.

The electronic device 200 may further display a suggestion provided based on the swing action parameter of the user. In this way, the user may learn of weakness of the user, and focus on improvement of the weakness during next training.

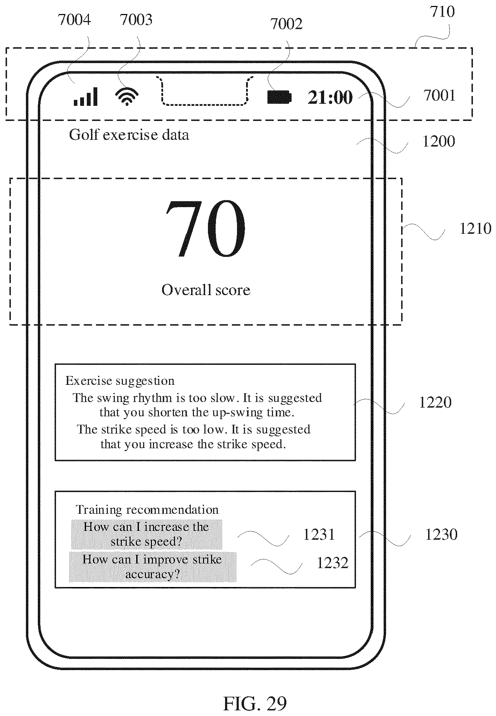

FIG. 29 is a diagram of an example UI of a user interface 1200 on which the electronic device 200 displays an exercise suggestion.

The user interface 1200 may include a score bar 1210 for an overall score, an exercise suggestion bar 1220, and a training recommendation bar 1230.

The score bar 1210 for the overall score includes an overall score of the user in one golf exercise. For example, the score may be 70.

The exercise suggestion bar 1220 includes some suggestions for the user in one golf exercise, for example, "The swing rhythm is too slow. It is suggested that you shorten the up-swing time." or "The strike speed is too low. It is suggested that you increase the strike speed." The exercise suggestion bar 1220 may further include more or less content. This is not limited in this application.

The training recommendation bar 1230 includes a recommended training course for a swing action parameter of the user, for example, a training course "How can I increase the strike speed?", and a training course "How can I improve strike accuracy?". The training recommendation bar 1230 may further include more or less content. This is not limited in this application.

It should be noted that the foregoing embodiments are merely used to describe this application, and should not be construed as a limitation.

A hardware architecture of the electronic device 200 mentioned in embodiments of this application is described below.

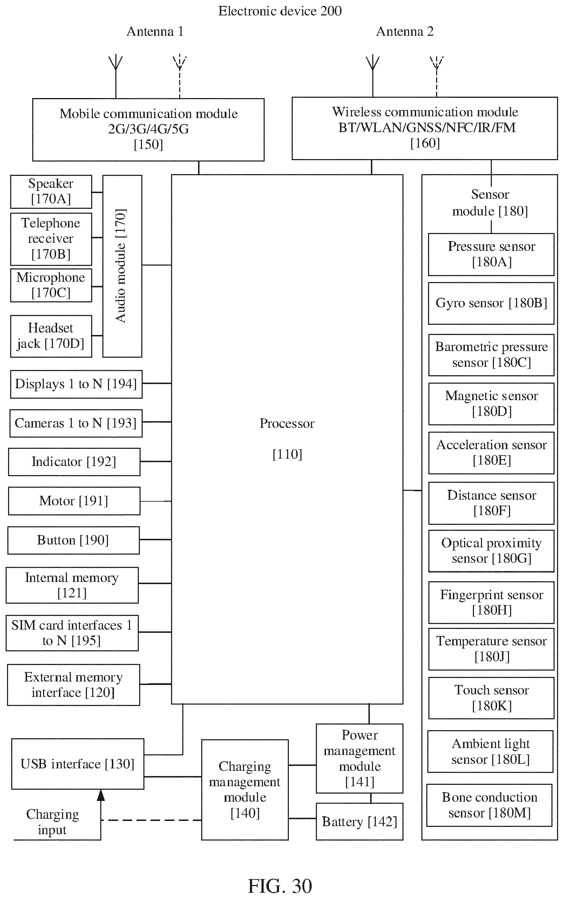

FIG. 30 is a schematic diagram of a structure of an electronic device 200.

The electronic device 200 is used as an example below to describe embodiments in detail. A device type of the electronic device 200 may include a mobile phone, a television, a tablet computer, a speaker, a watch, a desktop computer, a laptop computer, a handheld computer, a notebook computer, an ultra-mobile personal computer (ultra-mobile personal computer, UMPC), a netbook, a personal digital assistant (personal digital assistant, PDA), an augmented reality (augmented reality, AR)/virtual reality (virtual reality, VR) device, and the like. The device type of the electronic device 200 is not specially limited in this embodiment of this application.

It should be understood that the electronic device 200 shown in FIG. 30 is merely an example, and the electronic device 200 may have more or fewer components than those shown in FIG. 30, or two or more components may be combined, or there may be a different component configuration. Various components shown in the figure may be implemented in hardware that includes one or more signal processing and/or application-specific integrated circuits, software, or a combination of hardware and software.

The electronic device 200 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a telephone receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 200. In some other embodiments of this application, the electronic device 200 may include more or fewer components than those shown in the figure, or some components may be combined, or some components may be split, or there may be a different component layout. The components shown in the figure may be implemented by using hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, or a neural-network processing unit (neural-network processing unit, NPU). Different processing units may be independent devices, or may be integrated into one or more processors.

The controller may be a nerve center and a command center of the electronic device 200. The controller may generate an operation control signal based on an instruction operation code and a time sequence signal, to complete control of instruction fetching and instruction execution.

A memory may be further disposed in the processor 110, and is configured to store instructions and data. In some embodiments, the memory in the processor 110 is a cache. The memory may store instructions or data that has been used or is cyclically used by the processor 110. If the processor 110 needs to use the instructions or the data again, the processor may directly invoke the instructions or the data from the memory. This avoids repeated access, reduces a waiting time of the processor 110, and improves system efficiency.

In some embodiments, the processor 110 may include one or more interfaces. The interface may include an inter-integrated circuit (inter-integrated circuit, 12C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identification module (subscriber identity module, SIM) interface, a universal serial bus (universal serial bus, USB) interface, or the like.

The charging management module 140 is configured to receive a charging input from the charger. The charger may be a wireless charger or a wired charger. In some embodiments of wired charging, the charging management module 140 may receive a charging input from a wired charger through the USB interface 130. In some embodiments of wireless charging, the charging management module 140 may receive a wireless charging input through a wireless charging coil of the electronic device 200. The charging management module 140 may further supply power to the electronic device by using the power management module 141 while charging the battery 142.

The power management module 141 is configured to connect to the battery 142, the charging management module 140, and the processor 110. The power management module 141 receives an input of the battery 142 and/or the charging management module 140, and supplies power to the processor 110, the internal memory 121, an external memory, the display 194, the camera 193, the wireless communication module 160, and the like. The power management module 141 may be further configured to monitor parameters such as a battery capacity, a quantity of battery cycles, and a battery health status (electric leakage and impedance). In some other embodiments, the power management module 141 may alternatively be disposed in the processor 110. In some other embodiments, the power management module 141 and the charging management module 140 may alternatively be disposed in a same device.

A wireless communication function of the electronic device 200 may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 200 may be configured to cover one or more communication frequency bands. Different antennas may be further multiplexed, to improve antenna utilization.

The mobile communication module 150 may provide a solution applied to the electronic device 200 for wireless communication such as 2G/3G/4G/5G. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1.

The modem processor may include a modulator and a demodulator. The modulator is configured to modulate a to-be-sent low-frequency baseband signal into a medium- or high-frequency signal. The demodulator is configured to demodulate a received electromagnetic wave signal into a low-frequency baseband signal.

The wireless communication module 160 may provide a wireless communication solution that is applied to the electronic device 200 and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communication processor module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

The electronic device 200 may implement a display function by using the GPU, the display 194, the application processor, and the like. The display 194 is configured to display an image, a video, or the like. The display 194 includes a display panel.

The electronic device 200 may implement a photographing function by using the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image.

The camera 193 is configured to capture a static image or a video. An optical image of an object is generated by using the lens, and is projected onto the photosensitive element.

The digital signal processor is configured to process a digital signal, and may process another digital signal in addition to a digital image signal.

The NPU is a neural-network (neural-network, NN) computing processor. The NPU quickly processes input information with reference to a structure of a biological neural network, for example, a transfer mode between human brain neurons, and may further continuously perform self-learning.

The external memory interface 120 may be configured to connect to an external storage card, for example, a micro SD card, to extend a storage capability of the electronic device 200.

The internal memory 121 may be configured to store computer-executable program code. The executable program code includes instructions. The processor 110 implements various function applications and data processing of the electronic device 200 by running the instructions stored in the internal memory 121.

The electronic device 200 may implement an audio function, for example, music playing and recording, by using the audio module 170, the speaker 170A, the telephone receiver 170B, the microphone 170C, the headset jack 170D, the application processor, and the like.

The audio module 170 is configured to convert digital audio information into an analog audio signal output, and is also configured to convert an analog audio input into a digital audio signal. The audio module 170 may be further configured to encode and decode an audio signal.

The speaker 170A, also referred to as a "loudspeaker", is configured to convert an audio electrical signal into a sound signal. The electronic device 200 may listen to music or answer a hands-free call by using the speaker 170A.

The telephone receiver 170B, also referred to as an "earpiece", is configured to convert an electrical audio signal into a sound signal. When a call is answered or audio information is listened to by using the electronic device 200, the telephone receiver 170B may be put close to a human ear to listen to a voice.

The microphone 170C, also referred to as a "mike" or a "mic", is configured to convert a sound signal into an electrical signal. When making a call or sending speech information, a user may place the mouth of the user near the microphone 170C to make a sound, to input a sound signal to the microphone 170C. At least one microphone 170C may be disposed in the electronic device 200. In some other embodiments, two microphones 170C may be disposed in the electronic device 200, to collect a sound signal and further implement a noise reduction function. In some other embodiments, three, four, or more microphones 170C may alternatively be disposed in the electronic device 200, to collect a sound signal, implement noise reduction, and identify a sound source, to implement a directional recording function and the like.

In this embodiment, the electronic device 200 collects a sound signal by using the microphone 170C, and transmits the sound signal to an application on the electronic device 200.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 200 determines pressure intensity based on a capacitance change. When a touch operation is performed on the display 194, the electronic device 200 detects intensity of the touch operation based on the pressure sensor 180A. The electronic device 200 may calculate a touch location based on a detection signal of the pressure sensor 180A. In some embodiments, touch operations that are performed at a same touch location but have different touch operation intensity may correspond to different operation instructions. For example, when a touch operation whose touch operation intensity is less than a first pressure threshold is performed on a Messages icon, an instruction for viewing an SMS message is executed. When a touch operation whose touch operation intensity is greater than or equal to the first pressure threshold is performed on the Messages icon, an instruction for creating a new SMS message is executed.

The gyro sensor 180B may be configured to determine a motion posture of the electronic device 200. In some embodiments, an angular velocity of the electronic device 200 around three axes (that is, axes x, y, and z) may be determined by using the gyro sensor 180B. The gyro sensor 180B may be configured to implement image stabilization during photographing. For example, when the shutter is pressed, the gyro sensor 180B detects an angle at which the electronic device 200 jitters, calculates, based on the angle, a distance for which a lens module needs to compensate, and allows the lens to cancel the jitter of the electronic device 200 through reverse motion, to implement image stabilization. The gyro sensor 180B may be further used in a navigation scenario and a motion-sensing game scenario.

The barometric pressure sensor 180C is configured to measure barometric pressure. In some embodiments, the electronic device 200 calculates an altitude based on a value of the barometric pressure measured by the barometric pressure sensor 180C, to assist in positioning and navigation.

The magnetic sensor 180D includes a Hall sensor. The electronic device 200 may detect opening and closing of a flip cover by using the magnetic sensor 180D. In some embodiments, when the electronic device 200 is a clamshell phone, the electronic device 200 may detect opening and closing of a flip cover based on the magnetic sensor 180D.

Further, a feature such as automatic unlocking upon opening of the flip cover is set based on a detected opening or closing state of the flip cover.

The acceleration sensor 180E may detect accelerations in various directions (usually on three axes) of the electronic device 200. When the electronic device 200 is still, a magnitude and a direction of gravity may be detected. The acceleration sensor 180E may be further configured to identify a posture of the electronic device, and is used in an application such as switching between landscape mode and portrait mode or a pedometer.

The distance sensor 180F is configured to measure a distance. The electronic device 200 may measure a distance in an infrared manner or a laser manner. In some embodiments, in a photographing scenario, the electronic device 200 may measure a distance by using the distance sensor 180F, to implement quick focusing.

The optical proximity sensor 180G may include a light-emitting diode (LED) and an optical detector, for example, a photodiode. The light-emitting diode may be an infrared light-emitting diode. The electronic device 200 emits infrared light by using the light-emitting diode. The electronic device 200 detects infrared reflected light from a nearby object by using the photodiode. When sufficient reflected light is detected, the electronic device 200 may determine that there is an object near the electronic device 200. When insufficient reflected light is detected, the electronic device 200 may determine that there is no object near the electronic device 200. The electronic device 200 may detect, by using the optical proximity sensor 180G, that the user holds the electronic device 200 close to an ear for a call, to automatically turn off a screen for power saving. The optical proximity sensor 180G may also be used in a leather case mode or a pocket mode to automatically unlock or lock the screen.

The ambient light sensor 180L is configured to sense ambient light brightness. The electronic device 200 may adaptively adjust brightness of the display 194 based on the sensed ambient light brightness. The ambient light sensor 180L may also be configured to automatically adjust a white balance during photographing. The ambient light sensor 180L may also cooperate with the optical proximity sensor 180G to detect whether the electronic device 200 is in a pocket, to avoid an accidental touch.

The fingerprint sensor 180H is configured to collect a fingerprint. The electronic device 200 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 200 executes a temperature processing policy based on the temperature detected by the temperature sensor 180J. For example, when the temperature reported by the temperature sensor 180J exceeds a threshold, the electronic device 200 lowers performance of a processor near the temperature sensor 180J, to reduce power consumption and implement thermal protection. In some other embodiments, when the temperature is less than another threshold, the electronic device 200 heats the battery 142 to prevent the electronic device 200 from being shut down abnormally due to a low temperature. In some other embodiments, when the temperature is less than still another threshold, the electronic device 200 boosts an output voltage of the battery 142 to avoid abnormal shutdown due to a low temperature.

The touch sensor 180K may also be referred to as a touch panel or a touch-sensitive surface. The touch sensor 180K may be disposed on the display 194, and the touch sensor 180K and the display 194 form a touchscreen. The touch sensor 180K is configured to detect a touch operation performed on or near the touch sensor. The touch sensor may transfer the detected touch operation to the application processor to determine a type of a touch event. A visual output related to the touch operation may be provided by using the display 194. In some other embodiments, the touch sensor 180K may alternatively be disposed on a surface of the electronic device 200 at a location different from a location of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, and receive a blood pressure beating signal. In some embodiments, the bone conduction sensor 180M may alternatively be disposed in the headset, to constitute a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button or a touch button. The electronic device 200 may receive a key input, and generate a key signal input related to user settings and function control of the electronic device 200.

The motor 191 may generate a vibration prompt. The motor 191 may be configured to produce an incoming call vibration prompt and a touch vibration feedback. For example, touch operations performed on different applications (for example, photographing and audio playing) may correspond to different vibration feedback effects. For touch operations performed on different areas of the display 194, the motor 191 may also correspond to different vibration feedback effects. Different application scenarios (for example, time reminding, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a whiffed call, a notification, and the like.

The SIM card interface 195 is configured to connect to a SIM card. The SIM card may be inserted into the SIM card interface 195 or removed from the SIM card interface 195, to implement contact with or separation from the electronic device 200. The electronic device 200 may support one or N SIM card interfaces, where N is a positive integer greater than 1. The SIM card interface 195 may support a nano-SIM card, a micro-SIM card, a SIM card, and the like. A plurality of cards may be simultaneously inserted into a same SIM card interface 195. The plurality of cards may be of a same type or of different types. The SIM card interface 195 is compatible with different types of SIM cards. The SIM card interface 195 is also compatible with an external storage card. The electronic device 200 interacts with a network through the SIM card, to implement functions such as calling and data communication. In some embodiments, the electronic device 200 uses an eSIM, that is, an embedded SIM card. The eSIM card may be embedded into the electronic device 200, and cannot be separated from the electronic device 200.

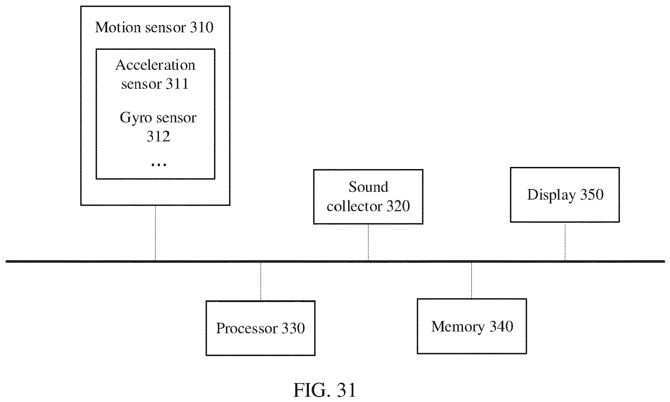

FIG. 31 is a schematic diagram of a structure of a wearable device 100. As shown in FIG. 31, the wearable device 100 may include a motion sensor 310, a sound collector 320, a processor 330, a memory 340, and a display 350 that are connected by using a bus.

The motion sensor 310 may be configured to collect first exercise data of a user within a first time period. The first exercise data may be exercise data of the wrist when the user plays golf, and may include one or more of the following: a moving distance in a vertical direction, a rotation angle, a speed of the wrist, and the like.

The motion sensor 310 may include an acceleration sensor 311 and a gyro sensor 312. After the wearable device 100 enables a golf mode, both the acceleration sensor 311 and the gyro sensor 312 are in an operating state. The acceleration sensor 311 may collect acceleration data of the wrist of the user, and the gyro sensor 312 may collect angular velocity data of the wrist of the user. The motion sensor 310 may send the acceleration data and the angular velocity data to the processor 330. The motion sensor 310 is not limited to the acceleration sensor 311 and the gyro sensor 312, and may further include more motion sensors. This is not limited in this application.

The sound collector 320 may be configured to collect a first sound signal within the first time period. The sound collector 320 may be a microphone or another apparatus configured to collect a sound signal. This is not limited in this embodiment of this application. After the wearable device 100 enables the golf mode, the sound collector 320 is in an operating state. The wearable device 100 may send the first sound signal to the processor 330.

The processor 330 may process the acceleration data and the angular velocity data to obtain an acceleration waveform feature and an angular velocity waveform feature, so as to calculate the moving distance of the wrist of the user in the vertical direction, the rotation angle, the speed of the wrist, and the like. The processor 330 may further determine, based on the first sound signal, an action (a whiff and a strike) of the user within the first time period. Specifically, the processor 330 may perform audio signal processing on the first sound signal to obtain a feature parameter of the first sound signal, so as to determine, by using a Gaussian mixture model in the processor 330, whether the first sound signal includes a sound signal generated by a strike.

The memory 340 may be configured to store a Gaussian mixture model, exercise data, and the like. The Gaussian mixture model may be a trained Gaussian mixture model.

The display 350 may be configured to display a user interface, such as a user interface including a control used to enable the golf mode, and a user interface including golf sports data.

It should be noted that the wearable device 100 further includes a touch panel coupled to the processor 330. The display 350 may display the user interface including the control used to enable the golf mode. When the touch panel detects a user operation (for example, tapping) performed on the control used to enable the golf mode, the processor 330 may determine whether the motion sensor 310 and the sound collector 320 are turned on. If the motion sensor 310 and the sound collector 320 are not turned on, the processor 330 may turn on the motion sensor 310 and the sound collector 320.

According to the context, the term "when" used in the foregoing embodiments may be interpreted as a meaning of "if" or "after" or "in response to determining" or "in response to detecting". Similarly, according to the context, the phrase "when it is determined that" or "if (a stated condition or event) is detected" may be interpreted as a meaning of "if it is determined that" or "in response to determining" or "when (a stated condition or event) is detected" or "in response to detecting (a stated condition or event)".

All or some of the foregoing embodiments may be implemented by using software, hardware, firmware, or any combination thereof. When software is used to implement embodiments, all or some of embodiments may be implemented in a form of a computer program product. The computer program product includes one or more computer instructions. When the computer program instructions are loaded and executed on a computer, all or some of the procedures or functions according to embodiments of this application are generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible to the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

Persons of ordinary skill in the art may understand that all or some of the procedures of the methods in embodiments may be implemented by a computer program instructing related hardware. The program may be stored in the computer-readable storage medium. When the program is executed, the procedures in the method embodiments may be included. The foregoing storage medium includes any medium that can store program code, such as a ROM, a random access memory RAM, a magnetic disk, or an optical disc.

**Claims**

1. A swing action detection method, applied to a wearable device, wherein the wearable device comprises one or more motion sensors and a sound collector, and the method comprises:

    collecting, by the wearable device, first exercise data by using the one or more motion sensors, wherein the first exercise data comprises acceleration data and angular velocity data;
    collecting, by the wearable device, a first sound signal by using the sound collector;
    determining, by the wearable device when the wearable device determines that the first sound signal meets a first condition, that a user action is a strike action;
    when the wearable device determines that the first exercise data meets a second condition, determining, by the wearable device, that a type of the strike action is a first strike action type; and
    displaying, by the wearable device, the first strike action type.

2. The method according to claim 1, wherein before the collecting, by the wearable device, first exercise data by using the one or more motion sensors, the method further comprises:

    displaying, by the wearable device, a first user interface, wherein the first user interface displays a first control; and
    detecting, by the wearable device, a first input performed on the first control; and
    the collecting, by the wearable device, first exercise data by using the one or more motion sensors specifically comprises:
    in response to the first input, collecting, by the wearable device, the first exercise data by using the one or more motion sensors.

3. The method according to claim 1, wherein before the collecting, by the wearable device, first exercise data by using the one or more motion sensors, the method further comprises:

    receiving, by the wearable device, a first instruction sent by an electronic device in response to a second input; and
    the collecting, by the wearable device, first exercise data by using the one or more motion sensors specifically comprises:
    in response to the first instruction, collecting, by the wearable device, the first exercise data by using the one or more motion sensors.

4. The method according to claim 1, wherein the method further comprises:

    determining, by the wearable device based on the first exercise data, an exercise parameter corresponding to the first strike action type, wherein the exercise parameter comprises one or more of the following: an up-swing time, a down-swing time, a swing rhythm, and a strike speed, and the swing rhythm is a ratio of the up-swing time to the down-swing time; and
    displaying, by the wearable device, a second user interface, wherein the second user interface comprises the exercise parameter corresponding to the first strike action type.

5. The method according to claim 1, wherein the method further comprises:

    determining, by the wearable device based on the first exercise data, an exercise parameter corresponding to the first strike action type, wherein the exercise parameter comprises one or more of the following: an up-swing time, a down-swing time, a swing rhythm, a strike speed, and a quantity of strikes, and the swing rhythm is a ratio of the up-swing time to the down-swing time; and

sending, by the wearable device to an electronic device, the exercise parameter corresponding to the first strike action type, wherein the exercise parameter corresponding to the first strike action type may be displayed on a third user interface displayed on the electronic device.

6. The method according to claim 1, wherein the user action comprises any one of the following: a strike action, a whiff action, and a ground hit action.

7. The method according to claim 1, wherein the determining, by the wearable device when the wearable device determines that the first sound signal meets a first condition, that a user action is a strike action specifically comprises:

calculating, by the wearable device, a first feature parameter of the first sound signal, wherein the first feature parameter comprises one or more of the following: an amount of energy, a frequency, and a peak value;

determining, by the wearable device, a first similarity based on the first feature parameter by using a first Gaussian mixture model, wherein the first similarity is used to indicate a similarity between the first feature parameter and a second feature parameter in the first Gaussian mixture model, and the second feature parameter comprises one or more of the following: an amount of energy, a frequency, and a peak value; and

when the first similarity is greater than a first threshold, determining, by the wearable device, that the user action is a strike action, wherein

the first condition comprises that the first similarity is greater than the first threshold.

8. The method according to claim 1, wherein the method further comprises:
determining, by the wearable device when the wearable device determines that the first sound signal meets the first condition and the first exercise data meets a third condition, that the user action is a strike action.

9. The method according to claim 8, wherein the determining, by the wearable device when the wearable device determines that the first sound signal meets the first condition and the first exercise data meets a third condition, that the user action is a strike action specifically comprises:

calculating, by the wearable device, a first feature parameter of the first sound signal, wherein the first feature parameter comprises one or more of the following: an amount of energy, a frequency, and a peak value;

determining, by the wearable device, a first similarity based on the first feature parameter by using a first Gaussian mixture model, wherein the first similarity is used to indicate a similarity between the first feature parameter and a second feature parameter in the first Gaussian mixture model, and the second feature parameter comprises one or more of the following: an amount of energy, a frequency, and a peak value;

determining, by the wearable device, an acceleration waveform feature based on the first exercise data; and

when the first similarity is greater than a first threshold and the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is greater than a first time threshold, determining, by the wearable device, that the user action is a strike, wherein

the first condition comprises that the first similarity is greater than the first threshold; and

the third condition comprises that the quantity of peaks or troughs in the acceleration waveform feature is greater than the first quantity threshold, the maximum peak value is greater than the first peak threshold, the difference between the maximum peak value and the minimum trough value adjacent to the maximum peak value is greater than the first difference threshold, and the time in which the maximum peak value decreases to the first acceleration threshold is greater than the first time threshold.

10. The method according to claim 6, wherein the wearable device determines, based on the first exercise data, that the user action is a strike action; and
that the wearable device determines, based on the first exercise data, that the user action is a strike action specifically comprises:

determining, by the wearable device, an acceleration waveform feature based on the first exercise data; and
when the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is greater than a first time threshold, determining, by the wearable device, that the user action is a strike, wherein

the first condition comprises that the quantity of peaks or troughs in the acceleration waveform feature is greater than the first quantity threshold, the maximum peak value is greater than the first peak threshold, the difference between the maximum peak value and the minimum trough value adjacent to the maximum peak value is greater than the first difference threshold, and the time in which the maximum peak value decreases to the first acceleration threshold is greater than the first time threshold.

11. The method according to claim 6, wherein the wearable device determines, based on the first exercise data, that the user action is a whiff action; and

that the wearable device determines, based on the first exercise data, that the user action is a whiff action specifically comprises:

determining, by the wearable device, an acceleration waveform feature based on the first exercise data; and when the wearable device determines that a quantity of peaks or troughs in the acceleration waveform feature is less than a first quantity threshold, a maximum peak value is less than a first peak threshold, a difference between the maximum peak value and a minimum trough value adjacent to the maximum peak value is less than a first difference threshold, and a time in which the maximum peak value decreases to a first acceleration threshold is greater than a first time threshold, determining, by the wearable device, that the user action is a whiff.

12. The method according to claim 6, wherein the wearable device determines, based on the first exercise data, that the user action is a ground hit action; and

that the wearable device determines, based on the first exercise data, that the user action is a ground hit action specifically comprises:

determining, by the wearable device, an acceleration waveform feature based on the first exercise data; and when the wearable device determines that a quantity of peaks or troughs in the acceleration waveform feature is greater than a first quantity threshold, a maximum peak value is greater than a first peak threshold, a difference between the maximum peak value and a minimum trough value adjacent to the maximum peak value is greater than a first difference threshold, and a time in which the maximum peak value decreases to a first acceleration threshold is less than a first time threshold, determining, by the wearable device, that the user action is a ground hit.

13. The method according to claim 1, wherein the first strike action type is a half-swing strike, and before the determining, by the wearable device, that a type of the strike action is a first strike action type, the method further comprises: determining, by the wearable device, a first rotation angle and/or a first moving distance of the wearable device within a first time based on the first exercise data, wherein the second condition is that the first rotation angle is less than or equal to a first preset angle and/or the first moving distance is less than or equal to a first preset distance within the first time.

14. The method according to claim 1, wherein the first strike action type is a full-swing strike, and before the determining, by the wearable device, that a type of the strike action is a first strike action type, the method further comprises: determining, by the wearable device, a first rotation angle and/or a first moving distance of the wearable device within a first time based on the first exercise data, wherein the second condition is that the first rotation angle is greater than a first preset angle and/or the first moving distance is greater than a first preset distance within the first time.

15. The method according to any one of claims 1 to 14, wherein the method further comprises:

counting, by the wearable device, a quantity of strikes within a second time period;
counting, by the wearable device, a quantity of whiffs and a quantity of ground hits within the second time period;
determining, by the wearable device, a strike rate based on the quantity of strikes within the second time period and the quantity of whiffs and the quantity of ground hits within the second time period; and
displaying, by the wearable device, a fourth user interface, wherein the fourth user interface comprises the strike rate.

16. A wearable device, comprising one or more processors, one or more memories, one or more motion sensors, a sound collector, and a display, wherein the one or more memories, the one or more motion sensors, the sound collector, and the display are coupled to the one or more processors, the one or more memories are configured to store computer program code, the computer program code comprises computer instructions, and the one or more processors invoke the computer instructions, so that the wearable device performs the method according to any one of claims 1 to 14.

17. A computer storage medium, comprising instructions, wherein when the instructions are run on a computer, the computer is enabled to perform the method according to any one of claims 1 to 14.

FIG. 1

Up-swing start point

FIG. 2

Up-swing stage

FIG. 3

Down-swing stage

FIG. 4

Finish stage

FIG. 5

Half swing

FIG.6

Full swing

FIG. 7

FIG. 8

FIG. 9

FIG. 10

FIG. 11

FIG. 12

FIG. 13

Enable the golf mode
An inertial sensor and a microphone need to be turned on to use this function. Do you want to turn on the inertial sensor and the microphone?

Confirm    Cancel

FIG. 14

EP 4 197 605 A1

The golf mode is enabled for the first time. Please input strike exercise data first. Do you want to input strike exercise data now?

Confirm    Cancel

FIG. 15

44

FIG. 16

120
110
111
119

Please select
duration for
inputting
personal data.

5 minutes    120
10 minutes   121
30 minutes   122

112

FIG. 17

FIG. 18

Inputting of
personal
exercise
data ends.

FIG. 19

FIG. 20

FIG. 21

7004    7003                7002                            710

Wearable device 100                                        900

Bluetooth  Connected                                       910

82%

Exercise data                      2201                    920

St                                              tance

1536    Data inputting is to                    m
        start after the band
        vibrates.
Sp

Golf mode          9301                          9304

Running            9302                          9305
mode
Swimming           9303                          9306
mode

930

FIG. 22

Wearable device 200

Gyro sensor     Acceleration sensor     Sound collector

S1001: Collect acceleration data, angular velocity data, and a sound wave signal

S1002: Obtain an acceleration waveform feature based on the acceleration data, obtain an angular velocity waveform feature based on the angular velocity data, and process the sound wave signal to obtain a feature parameter of the sound wave signal

S1003: Determine, through analysis based on the acceleration waveform feature and the angular velocity waveform feature, that a user action is any one of a whiff, a ground hit, and a strike

S1004: Perform similarity matching calculation on the feature parameter of the sound wave signal and a Gaussian mixture model, and further determine that the sound wave signal is generated by a strike

S1005: After determining a strike action, the wearable device 100 calculates a swing action parameter based on the acceleration waveform feature and the angular velocity waveform feature, and calculates a quantity of strikes based on the sound wave signal generated by the strike

S1006: Classify golf actions of a user into an effective strike action and an ineffective strike action

FIG. 23

FIG. 24A

FIG. 24B

**Ground hit**

FIG. 24C

FIG. 25

FIG. 26

EP 4 197 605 A1

FIG. 27

58

7004   7003        7002                              710

.ıll  📶          📶  21:00              7001

Golf exercise data                                  1100

Quantity of effective strikes      10 strikes

Full-swing up-swing time                            1110

Average up-                  Maximum up-
swing time        600 ms     swing time     650 ms

ms                                                  1111

Full-swing down-swing time                          1120

Average down-                Maximum down-
swing time        200 ms     swing time     230 ms

ms                                                  1121

Full-swing rhythm                                   1130

3                                                   1131

FIG. 28

7004   7003            7002                    710

.ıll  🛜  [.............]  ▬ 21:00         7001

Golf exercise data                          1200

70

Overall score                              1210

Exercise suggestion
  The swing rhythm is too slow. It is suggested
      that you shorten the up-swing time.
  The strike speed is too low. It is suggested
      that you increase the strike speed.        1220

Training recommendation
  How can I increase the
      strike speed?              1231        1230
  How can I improve strike
      accuracy?                  1232

FIG. 29

Electronic device 200

Antenna 1                                    Antenna 2

| Mobile communication module 2G/3G/4G/5G [150] | Wireless communication module BT/WLAN/GNSS/NFC/IR/FM [160] |

Speaker [170A]

Telephone receiver [170B]

Microphone [170C]

Headset jack [170D]

Audio module [170]

Displays 1 to N [194]

Cameras 1 to N [193]

Indicator [192]

Motor [191]

Button [190]

Internal memory [121]

SIM card interfaces 1 to N [195]

External memory interface [120]

Processor

[110]

Sensor module [180]

Pressure sensor [180A]

Gyro sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

USB interface [130]

Charging input

Charging management module [140]

Power management module [141]

Battery [142]

FIG. 30

Motion sensor 310

Acceleration
sensor 311

Gyro sensor
312

...

Sound
collector 320

Display 350

Processor 330

Memory 340

FIG. 31

## INTERNATIONAL SEARCH REPORT

| International application No. |
|---|
| **PCT/CN2021/116068** |

**A.    CLASSIFICATION OF SUBJECT MATTER**

A63B 69/36(2006.01)i

According to International Patent Classification (IPC) or to both national classification and IPC

**B.    FIELDS SEARCHED**

Minimum documentation searched (classification system followed by classification symbols)

A63B69/-; G06F

Documentation searched other than minimum documentation to the extent that such documents are included in the fields searched

Electronic data base consulted during the international search (name of data base and, where practicable, search terms used)

CNTXT, CNABS, CNKI, VEN: 挥杆, 击球, 高尔夫, 羽毛球, 网球, 穿戴, 手环, 手表, 传感器, 声音, 震动, 振动, 高斯; swing, hit, golf, badminton, tennis, wear, bracelet, watch, sensor, sound, shock, vibration, Gauss

**C.    DOCUMENTS CONSIDERED TO BE RELEVANT**

| Category* | Citation of document, with indication, where appropriate, of the relevant passages | Relevant to claim No. |
|---|---|---|
| X | CN 106843490 A (GUANGDONG XIAOTIANCAI TECHNOLOGY CO., LTD.) 13 June 2017 (2017-06-13)<br>description paragraphs 48-134, figures 1-5 | 1-3, 6, 8, 15-17 |
| A | CN 203763810 U (BEIJING NOITOM TECHNOLOGY LTD.) 13 August 2014 (2014-08-13)<br>entire document | 1-17 |
| A | CN 110633103 A (CASIO COMPUTER CO., LTD.) 31 December 2019 (2019-12-31)<br>entire document | 1-17 |
| A | WO 2016208794 A1 (PARK WON IL) 29 December 2016 (2016-12-29)<br>entire document | 1-17 |
| A | KR 20130124836 A (ONBIT INC et al.) 15 November 2013 (2013-11-15)<br>entire document | 1-17 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 November 2021** | **02 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/116068**

| Patent document cited in search report | | | Publication date (day/month/year) | Patent family member(s) | | | Publication date (day/month/year) |
|---|---|---|---|---|---|---|---|
| CN | 106843490 | A | 13 June 2017 | None | | | |
| CN | 203763810 | U | 13 August 2014 | None | | | |
| CN | 110633103 | A | 31 December 2019 | JP | 2019220046 | A | 26 December 2019 |
| | | | | JP | 6787368 | B2 | 18 November 2020 |
| | | | | US | 2019388727 | A1 | 26 December 2019 |
| WO | 2016208794 | A1 | 29 December 2016 | KR | 20170001206 | A | 04 January 2017 |
| | | | | KR | 101707319 | B1 | 15 February 2017 |
| KR | 20130124836 | A | 15 November 2013 | None | | | |

Form PCT/ISA/210 (patent family annex) (January 2015)

**EP 4 197 605 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010910396